# EUROPEAN PATENT SPECIFICATION

(11) **EP 3 745 406 B1**
(45) Date of publication and mention of the grant of the patent: **03.09.2025**
(21) Application number: 19184695.5
(22) Date of filing: 05.07.2019
(51) Int. Cl.: G16B 20/20, G16B 30/10

(54) **SYSTEM AND METHOD FOR PREDICTING EFFECT OF GENOMIC VARIATIONS ON PRE-MRNA SPLICING**
SYSTEM UND VERFAHREN ZUR VORHERSAGE DER WIRKUNG VON GENOMISCHEN VARIATIONEN BEIM PRE-MRNA-SPLEISSEN
SYSTÈME ET PROCÉDÉ PERMETTANT DE PRÉDIRE L'EFFET DES VARIATIONS GÉNOMIQUES SUR L'ÉPISSAGE PRÉ-ARNM

(30) Priority: 07.07.2018 IN 201821025433
(43) Date of publication of application: 02.12.2020
(73) Proprietor: Tata Consultancy Services Limited, Maharashtra (IN)
(72) Inventor: SRINIVASAN, RAJGOPAL, 500034 Hyderabad, Telangana (IN); JAIN, Akriti, Hyderabad - 500034 Telangana (IN); CHAUDHURI, Poulami, 500034 Hyderabad, Telangana (IN)
(74) Representative: Goddar, Heinz J.

(56) References cited:
- WO-A1-2017/220315
- US-A1- 2014 199 698
- US-A1- 2017 240 900
- ANONYMOUS: "In silico tools for splicing defect prediction - A survey from the viewpoint of end-users", 31 July 2014 (2014-07-31), XP055740912, Retrieved from the Internet <URL:https://www.ncbi.nlm.nih.gov/pmc/articles/PMC4029872/> [retrieved on 20201016]
- JIA WEN ET AL: "A heuristic model for computational prediction of human branch point sequence", BMC BIOINFORMATICS, BIOMED CENTRAL LTD, LONDON, UK, vol. 18, no. 1, 24 October 2017 (2017-10-24), pages 1 - 9, XP021250154, DOI: 10.1186/S12859-017-1864-9
- ANONYMOUS: "Quick Start Guide for Phytozome v10.1", 29 July 2016 (2016-07-29), XP055740798, Retrieved from the Internet <URL:https://web.archive.org/web/20160729232506/https://phytozome.jgi.doe.gov/pz/QuickStart.html> [retrieved on 20201016]

## Description

### Technical Field

The disclosure herein generally relates to mRNA splicing, and, more particularly, predicting effect of genomic variations on pre-mRNA splicing.

### Background

RNA splicing is a process of cutting introns out of pre-mRNA and stitching together exons to form a final nucleotide sequence that is the mRNA sequence that codes for proteins. In this regard branchpoint (BP) selection and splice site (SS) selection are key steps in RNA splicing, yet many popular splicing analysis tools do not model this mechanism. If there is a mutation in proximity to an intron's primary branch point, that branchpoint may become unusable.

Existing methods for branchpoint prediction use wet lab techniques and in-silico methods. The wet lab techniques are time consuming and labour intensive, while existing computational models involving Support Vector Machine algorithm or machine learning tools are based on numerous assumptions which hamper accurate prediction. Various computational methods have been implemented to facilitate accurate branchpoint prediction and the predicted branchpoints have been tested in vivo/vitro but most of the models are built on hypothetical assumptions which do not lead to accurate prediction of branchpoints. In general the search for disease-causing mutations has been mostly restricted to coding exons, intron-exon junction and promoter region of the gene of interest.

Anonymous: "In silico tools for splicing defect prediction: a survey from the viewpoint of end users" discloses RNA splicing is the process during which introns are excised and exons are spliced. The precise recognition of splicing signals is critical to this process, and mutations affecting splicing comprise a considerable proportion of genetic disease etiology. Analysis of RNA samples from the patient is the most straightforward and reliable method to detect splicing defects. However, currently, the technical limitation prohibits its use in routine clinical practice. In silico tools that predict potential consequences of splicing mutations may be useful in daily diagnostic activities. In this review, we provide medical geneticists with some basic insights into some of the most popular in silico tools for splicing defect prediction, from the viewpoint of end users. Bioinformaticians in relevant areas who are working on huge data sets may also benefit from this review. Specifically, focus on those tools whose primary goal is to predict the impact of mutations within the 5' and 3' splicing consensus regions: the algorithms used by different tools as well as their major advantages and disadvantages are briefly introduced.

### SUMMARY

Embodiments of the present disclosure present technological improvements as solutions to one or more of the above-mentioned technical problems recognized by the inventors in conventional systems. The scope of the invention is defined by the appended claims.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG.1 illustrates network environment implementing a system 102 for predicting effect of genomic variations on pre-mRNA splicing, in accordance with an embodiment of the present disclosure.
FIG.2 is a flow diagram illustrating a method for predicting effect of genomic variations on pre-mRNA splicing, according to an embodiment of the present disclosure.
FIG. 3A-3C illustrates an analysis pipeline for predicting effect of genomic variations on pre-mRNA splicing, in accordance with an embodiment of the present disclosure.
FIG. 4 illustrates a block diagram of a system for predicting effect of genomic variations on pre-mRNA splicing, in accordance with embodiments of the present disclosure

### DETAILED DESCRIPTION

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the claims (when included in the specification).

One of the study for investigating disease-causing BPS mutations provides that in adenosine branchpoints in comparison to other base branchpoints caused more severe splicing defects. A mutation in the branchpoint impairs the lariat formation and may lead to aberrant splicing of the intron, leading to gene dysfunction. The lariat is a lasso-shaped structure formed during the removal of introns in mRNA processing. Mutations at branch sites have been shown to lead to aberrant splicing, which in turn can lead to disease phenotypes. The explosion of the use of next generation sequencing (NGS) in the clinic for diagnosis and screening of disorders may benefit from approaches that can reliably identify mutations in branch sites that may be explanatory of diseases. Development of such tools has been hampered by the absence of a large enough "gold dataset" of known high confident branch sites.

Splicing forms a crucial part of pre-mRNA maturation process as accurate excision of introns and joining of exons are essential to eukaryotic gene expression. During splicing, parts of the pre-mRNA are removed by the spliceosome within the nucleus before the mature mRNA is transported to the cytoplasm for translation. Depending upon tissue localization and the developmental stage, pre-mRNA is differently spliced leading to alternative transcripts i.e., expression of different proteins from the same gene. More than 70% of protein coding human gene are alternatively spliced and alternative splicing has been proposed to be the major cause of the evolution of phenotypic complexity in mammals.

Exon skipping is the most common outcome of splicing mutations, followed by activation of cryptic 5' and 3' splice sites (5'SS and 3'SS). Exon skipping is due to disruption of natural splice acceptor site or abolishment of the natural branchpoint with no alternative branchpoint available to facilitate splicing. Efficient splicing requires at least three major signals within introns, the 5' splice site, 3' splice site and the branchpoint sequence. Auxiliary sequences in introns and exons known as splicing enhancers and silencers act in conjunction to decide splicing to be constitutive or alternative. The 5' end of the intron is known as splice donor site and 3' end of the intron is referred as splice acceptor site.

The divergence from the prototype sequences are associated with alternative transcript generation. Occurrence of such consensus sequences within the introns is quite common in the case of higher eukaryotes framing pseudoexons, indicating the presence of the splice boundaries but insufficient for regulating correct splicing. The 3' end is characterized by presence of the splice acceptor site, branchpoint sequence upstream and the polypyrimidine tract immediately following the branchpoint sequence. Branchpoints are defined on the basis of four major criteria: that are proximal to the 3' splice end of the intron, branchpoint sequence is followed by polypyrimidine tract, a depletion of 'AG' dinucleotide between the branchpoint sequence and the 3' splice site, and the branchpoint is mostly an adenine. So the selection and accurate prediction of branchpoint variant and splice site variant from candidate variants of existing databases of known human gene transcripts is of prime importance and challenging.

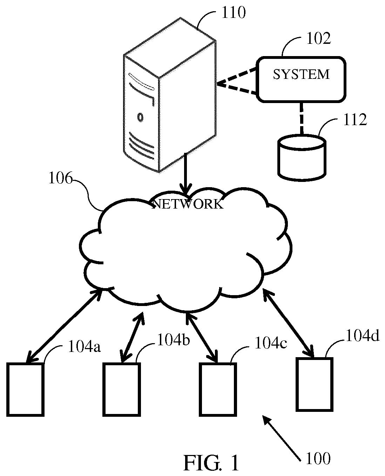

Various embodiments of the present disclosure provided method and system for predicting the effect of genomic variations on pre-mRNA splicing based on MaxEnt tool and a Position Weight Matrix (PWM) evaluator with high accuracy utilized on resource constrained environment. The disclosed system includes a variant pipeline which works in real-time in a resource constrained environment or near real-time on CPU. The disclosed system and method provides a solution in predicting effect of genomic variations on pre-mRNA splicing. A detailed description of the above described system and method for predicting the effect of genomic variations on pre-mRNA splicing is shown with respect to illustrations represented with reference to FIGS. 1 through 4.

Referring now to the drawings, and more particularly to FIGS. 1 through 4, where similar reference characters denote corresponding features consistently throughout the figures, there are shown preferred embodiments and these embodiments are described in the context of the following exemplary system and method for predicting effect of genomic variations on pre-mRNA splicing.

Herein, the system 102 may receive inputs, for example, inputs via multiple devices and/or machines 104-1, 104-2... 104-N, collectively referred to as devices 104 hereinafter. Examples of the devices 104 may include, but are not limited to, a portable computer, a personal digital assistant, a handheld device, VR camera embodying devices, storage devices equipped to receive and store inputs and outputs. In an embodiment, the devices 104 may include devices capable of capturing and storing data. The devices 104 are communicatively coupled to the system 102 through a network 106, and may be capable of transmitting the data to the system 102.

In one implementation, the network 106 may be a wireless network, a wired network or a combination thereof. The network 106 can be implemented as one of the different types of networks, such as intranet, local area network (LAN), wide area network (WAN), the internet, and the like. The network 106 may either be a dedicated network or a shared network. The shared network represents an association of the different types of networks that use a variety of protocols, for example, Hypertext Transfer Protocol (HTTP), Transmission Control Protocol/Internet Protocol (TCP/IP), Wireless Application Protocol (WAP), and the like, to communicate with one another. Further the network 106 may include a variety of network devices, including routers, bridges, servers, computing devices, storage devices, and the like.

The devices 104 send input to the system 102 via the network 106. The system 102 is caused to predict effect of genomic variations on pre-mRNA splicing. In an embodiment, the system 102 may be embodied in a computing device 110. Examples of the computing device 110 may include, but are not limited to, a desktop personal computer (PC), a notebook, a laptop, a portable computer, a smart phone, a tablet, and the like. The system 102 may also be associated with a data repository 112 to store inputs, dataset and output/resultant. Additionally or alternatively, the data repository 112 may be configured to store data and/or information generated during predicting effect of genomic variations on pre-mRNA splicing. The repository 112 may be configured outside and communicably coupled to the computing device 110 embodying the system 102. Alternatively, the data repository 112 may be configured within the system 102.

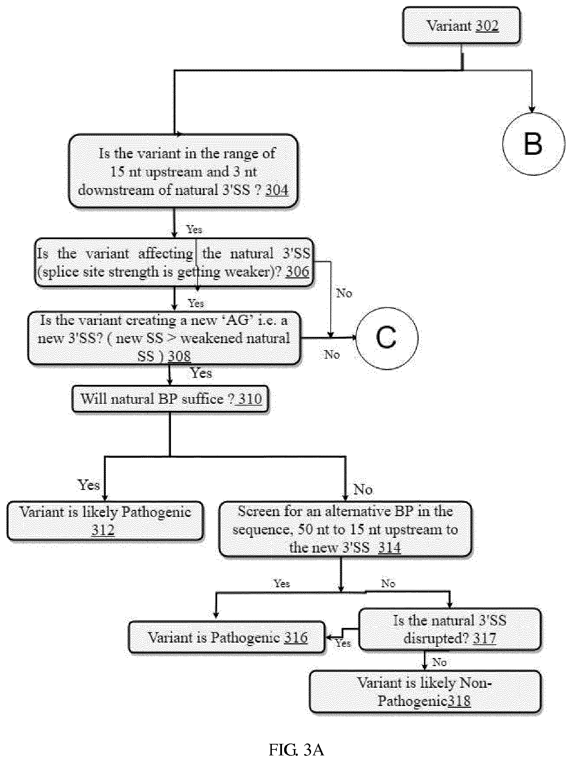

In an embodiment, the disclosed system 102 enables predicting effect of genomic variations on pre-mRNA splicing, thereby resulting in high accuracy of predicting pathogenicity and determining branchpoint variants and their pathogenicity based on the availability of alternative branchpoint which could rescue normal splicing. An example representation of pipeline of the method for predicting effect of genomic variations on pre-mRNA splicing is shown and described further with reference to FIG. 3A-3C.

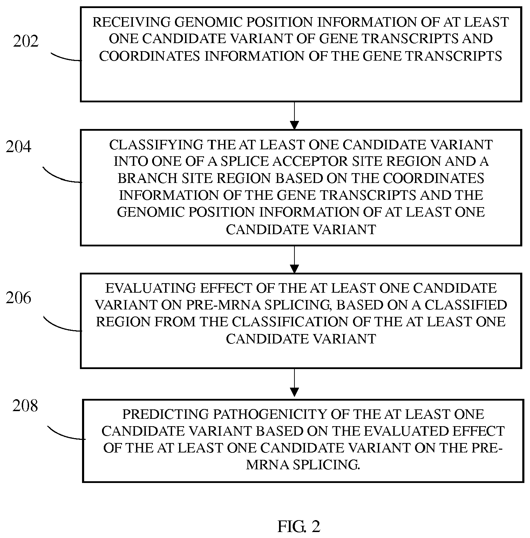

Referring now to FIG. 2, a flow-diagram of a method 200 for predicting effect of genomic variations on pre-mRNA splicing is described, according to embodiments of present disclosure. The method 200 may be described in the general context of computer executable instructions. Generally, computer executable instructions can include routines, programs, objects, components, data structures, procedures, modules, functions, etc., that perform particular functions or implement particular abstract data types. The method 200 may also be practiced in a distributed computing environment where functions are performed by remote processing devices that are linked through a communication network. The order in which the method 200 is described is not intended to be construed as a limitation, and any number of the described method blocks can be combined in any order to implement the method 200, or an alternative method. Furthermore, the method 200 can be implemented in any suitable hardware, software, firmware, or combination thereof. In an embodiment, the method 200 depicted in the flow chart may be executed by a system, for example, the system 102 of FIG. 1. In an example embodiment, the system 102 may be embodied in an exemplary computer system, for example computer system 102. The method 200 of FIG. 2 will be explained in more detail below with reference to FIGS. 3A-3C.

Referring to FIG. 2, in the illustrated embodiment, the method 200 is initiated at 202 where genomic position information, reference allele and alternate allele corresponding to a specified version of the human genome is received. The at least one candidate transcript that fully contains the variant is obtained from existing databases of known human gene transcripts (herein, referred to as at least one variant). Each transcript is represented as a set of one or more non-overlapping intervals, where each interval is represented by four features that include the chromosome on which the transcript is present, the starting genomic coordinate of the interval, the ending genomic coordinate of the interval, and the strand on which the transcript is present (forward or reverse).

At 204 the at least one candidate variant is classified as occurring in one of a splice acceptor site region and a branch site region based on the coordinates information of the gene transcripts and the genomic position information of the at least one candidate variant. Further, the at least one candidate variant is classified as the splice acceptor site region occurring in genomic coordinate between 15 nucleotides upstream to 3 nucleotides downstream of a natural intron-exon splice acceptor junction of the gene transcripts and as the branch site region occurring in genomic coordinate between 50 nucleotides to 15 nucleotides upstream of a natural splice acceptor junction of the gene transcripts. Herein nucleotide and nt and used interchangeable.

At 206, effect of the at least one candidate variant on pre-mRNA splicing is evaluated based on a classified region from the classification of the at least one candidate variant. The evaluation is performed by identifying weakening of a natural splice acceptor site in the classified region, due to the at least one candidate variant, using a MaxEnt score and then determining that a new splice acceptor site region is being created due to the weakened natural splice acceptor site. Thereafter in response to determining that the new splice acceptor site region being created, strength of an identified natural branch point in the classified region using a Position Weight Matrix (PWM) evaluator. The MaxEnt is a known splice site strength determination tool for calculating strength or weakening of the splice acceptor site, wherein the MaxEnt tool assigns a MaxEnt score based on the effect of the at least one candidate variant on affected natural splice acceptor site region. In an example embodiment, the available MaxEnt Scan tool is used to calculate the splice acceptor site scores for both the canonical splice sites which is the natural occurring splice sites or natural splice site acceptor region and cryptic splice sites which is splice sites activated by a mutation.

The PWM evaluator is generated using experimentally determined human branch sites. In an example embodiment, the PWM is generated using an experimentally determined 59,359 human branch sites (10 mers), identified based on exoribonuclease digestion and RNA-seq. In said example embodiment, a set of branch point sites is utilized by selecting only the sequences with 'A' at the branchpoint as the training set for the Position weight matrix (PWM). In said example embodiment, 'A' is chosen as the branchpoint since ones with 'C'/'T'/G as the branchpoint has very low median scores, while the known A has the highest value, suggesting the PWM generated, in accordance with present embodiments, has a selectivity towards 'A' as a branchpoint and is ideal to restrict the PWM scoring to 'A'. Therefore the PWM was built using the known 'A' as the branchpoint. A PWM matrix of (m*n) is created by aligning the experimentally determined 59,359 human branch sites (10 mers) with 'A' as the branchpoint. In present embodiment a matrix of (10*4) is created. The alignment is then used in calculating the frequency of each nucleotide at each position of the 10mers and thereafter the frequencies of each nucleotide are converted to log odds scores.

In said example embodiments, 1,75,031 unique introns from 18,171 canonical transcripts from Gencode database v19 is identified and extracted with the filtering criterion of being surrounded by coding exons on both sides. The frequency of each nucleotide (A, T, C, G) across all the introns is used to normalize the raw frequencies of the bases in the training set of branch points. As described above, the normalized frequencies are converted to log odds scores to generate the final PWM. Based on the branch site scores obtained for the known branch sites with 'A' as the branchpoint. The first quartile of the distribution is calculated and is used as a threshold for classifying a site to be a high confidence branch site. In an example embodiment, the determined threshold is 1.46. Further, a 40 mer intronic sequence, 10 to 50 bases upstream from the 3' end of each intron is extracted from the human genome and scanned for 10 mer sequences scoring above the branchpoint threshold.

At 208, pathogenicity of the at least one candidate variant is predicted based on the evaluated effect of the at least one candidate variant on the pre-mRNA splicing. Further evaluation and predicting pathogenicity of the at least one candidate variant is further described in detail in reference to FIGS 3A-3C.

Referring now to FIGS. 3A-3C, illustrating the analysis pipeline for method of predicting pathogenicity on the pre-mRNA splicing. Herein the analysis pipeline is designed to categorize a variant as pathogenic or non-pathogenic. The analysis approach, in accordance with the present embodiments follows a step by step pipeline represented by FIGS 3A-3C. In an embodiment, variants that were in close proximity, that is up to 15 nucleotide upstream to the canonical splice acceptor region are screened for creation of a new cryptic acceptor site or a creation of a new branch site. If a branch site is created, then a suitable downstream splice acceptor site scan is initiated. If the variant is creating a splice acceptor, then a suitable upstream branch site is scanned for using the PWM evaluator. If the variant disrupted the canonical splice acceptor and the canonical branch site is unaffected, then the screening for a suitable alternative downstream splice acceptor is performed. If a new canonical splice acceptor was predicted downstream to the canonical splice acceptor site, then a screening for a experimentally proven branchpoint is performed using the PWM tool. The detailed step by step process of the pipeline is described in FIGS. 3A-3C.

Referring now to FIG. 3A, where a variant 302, for example, at least one candidate variant is received where genomic position information, reference allele and alternate allele corresponding to a specified version of the human genome is received. The at least one candidate transcript that fully contains the variant is obtained from existing databases of known human gene transcripts (herein, referred to as at least one variant). Each transcript is represented as a set of one or more non-overlapping intervals, where each interval is represented by four features that include the chromosome on which the transcript is present, the starting genomic coordinate of the interval, the ending genomic coordinate of the interval, and the strand on which the transcript is present (forward or reverse). The at least one candidate variant is classified as occurring in splice affecting region based on genomic coordinate. At 304, region occurring in genomic coordinate between 15 nucleotides upstream to 3 nucleotides downstream of a natural intron-exon splice acceptor junction of the gene transcripts is classified as splice acceptor site. At 306, weakening of the natural splice acceptor site is determined and, in other words, it is determined that the classified at least one candidate variant is affecting the natural splice acceptor site (natural 3'SS). At 308, the classified at least one candidate variant is checked for creating a new 'AG' that is a new 3'SS thereby weakening of the natural 3'SS as determined using MaxEnt score. In response to the determined weakening of the splice acceptor site, that is the weakening of the natural 3'SS, the at least one candidate variant is checked if natural branchpoint suffices or branches out to block C. In other words, at 310, determining presence or absence of natural branchpoint in sequence range of 15 nucleotide to 50 nucleotide of the new splice acceptor site region being active during the pre-mRNA splicing. Thereafter strength of the natural branchpoint is evaluated using the PWM evaluator and identifying the at least one candidate variant as pathogenic (312) based on the evaluated strength of the natural branchpoint; or screening for an alternative branchpoint using the PWM evaluator and predicting the at least one candidate as a pathogenic based on the evaluated strength of the alternative branchpoint (314). At 317, status of the natural splice acceptor site region is determined. The status herein includes disrupted natural splice acceptor site region or non-disrupted natural splice acceptor site region. At 316, the at least one candidate variant is predicted as pathogenic or non-pathogenic (318) based on the determined status.

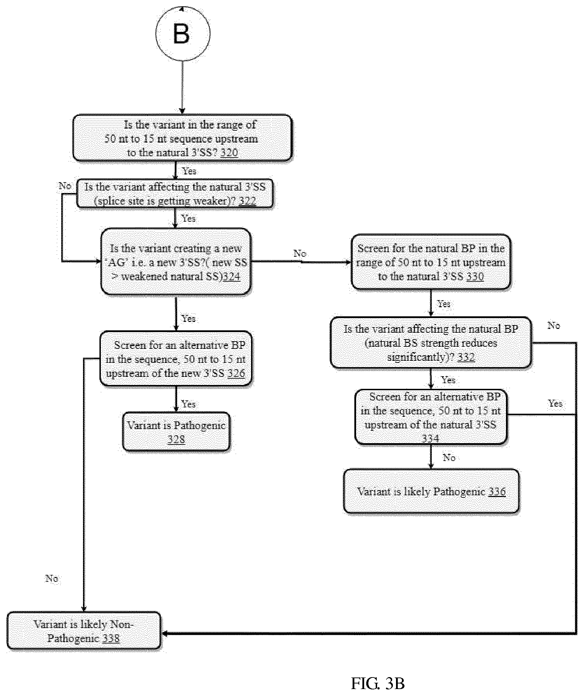

Referring now to FIG. 3B, at connector B, the at least one candidate variant is classified as occurring in branch site region based on genomic coordinate. At 320, region with genomic coordinate between 50 nucleotides to 15 nucleotides upstream of a natural splice acceptor junction of the gene transcripts is classified as branch site. At 322, weakening of the natural splice acceptor site is determined and, in other words, it is determined that the classified at least one candidate variant is affecting the natural 3'SS. At 324 the classified at least one candidate variant is checked for creating a new 'AG' that is a new 3'SS thereby weakening of the natural 3'SS in response to the creation of the new 3'SS is determined using MaxEnt score. In response to the determined weakening of the splice acceptor site is screened either for natural branchpoint or alternative branchpoint. At 326, the effect of the at least one candidate variant on the branch site for the new splice acceptor site being created is evaluated by determining presence of an alternative branchpoint in sequence range 50 nucleotides to 15 nucleotides upstream of the new splice acceptor site. At, 328 the at least one variant is categorized to be pathogenic if no alternative branchpoint is determined, at 338 the at least one candidate variant is predicted as non-pathogenic if an alternative branchpoint is found.

At 330, the effect of the at least one candidate variant on the branch site for no new splice acceptor site being created is evaluated by screening for natural branchpoint in sequence range having 50 nucleotides to 15 nucleotides upstream of the natural splice acceptor site and determining level of strength of the branch site using the PWM evaluator at 332. Herein, the level of strength is determined due to the at least one candidate variant affecting the screened natural branchpoint. At 334, based on the determined level of strength of the branch site the at least one candidate variant is predicted as pathogenic. At 336, the at least one candidate variant is predicted as pathogenic or non-pathogenic (338) based on an alternative branchpoint screened in sequence range of 50 nucleotides to 15 nucleotides upstream of the natural splice acceptor site region.

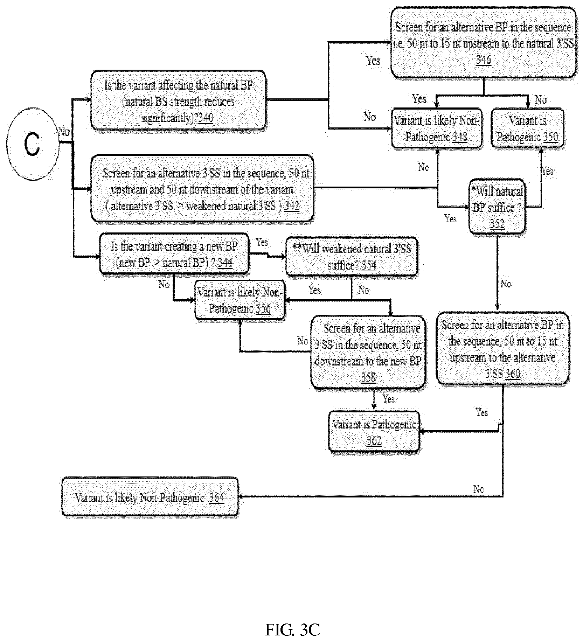

Referring now to FIG. 3C, at connector C, effect of the at least one candidate variant on the splice acceptor site region for no new splice acceptor site being created is evaluated by sequentially performing the steps at 340, 342 and 344. At 340 effect of the at least one candidate variant on the natural branchpoint is determined and level of strength of natural branch site using the PWM evaluator is identified based on the determined effect. At 342, for an alternative splice acceptor site region in sequence range having 50 nucleotide upstream and 50 nucleotide downstream of the at least one candidate variant is screened and a comparison of strength of the alternative splice acceptor site region and weakened natural splice acceptor site region is performed. At 344, presence of a new branchpoint being created and performing comparison of strength of the new branchpoint and the natural branchpoint is determined. Further based on 340, at 346, the at least one candidate variant is predicted as a non-pathogenic variant (348) or the at least one variant candidate is predicted as a pathogenic variant (350) or a non-pathogenic variant (364) based on a screened alternative branchpoint (360) in the sequence 50 nucleotide to 15 nucleotide upstream to the natural splice acceptor site region.

Further based on 342, the at least one candidate variant is predicted as non-pathogenic (348) or further presence of natural branchpoint in sequence range of 15 nucleotide to 50 nucleotide to the splice acceptor site region being active during the mRNA splicing is determined (352) and thereafter strength of the natural branchpoint with the predefined threshold is compared. And, based on the comparison the at least one candidate variant is predicted as pathogenic (350). Further based on 344, the at least one candidate variant is predicted as pathogenic (354) or non-pathogenic (356) based on an alternative branchpoint screened in the sequence range of 50 nucleotides to 15 nucleotides upstream of the alternative splice acceptor site (358). Further, based on the comparison of strength of the new branchpoint and the natural branchpoint, the at least one candidate variant is predicted as non-pathogenic (364). If not, presence of natural branchpoint in the range of 15 nucleotide to 50 nucleotide upstream to the splice acceptor site region being active during the mRNA splicing is determined and thereafter strength of the natural branchpoint with the predefined threshold (354). Based on the determined presence of natural branchpoint and comparison of strength of the natural branchpoint with the predefined threshold the at least one candidate variant is predicted as pathogenic (362) or non-pathogenic (364).

In accordance with the present embodiments, the focus of the present system and method is to identify a BP given at a random sequence and evaluate the identified BP's role in the functional consequence of splicing of the intron. Further the focus of the present embodiments to predict the impact of the evaluated BP on pathogenicity using a combination of PWM and MaxEnt score. There are many tools which can predict a branchpoint, but the main drawback is it requires far more input data while predicting BP, like the polypyramidine tract information, the actual splice acceptor site and the distance to the splice acceptor site region, which restricts such tools to predict a branchpoint given at a random sequence. The present system and method clearly distinguishes between the BP and SS and evaluates a variant based on the combined output from an individual component.

### VALIDATION AND RESULTS

The results of methods for predicting effect of genomic variations on pre-mRNA splicing have been validated using following examples. It will be understood that the examples discussed herein are only for the purpose of explanation and not to limit the scope of the present subject matter. Further, the test results are shown for a specific example of predicting effect of genomic variations on pre-mRNA splicing and should in no way be construed as the only method that can be formed through the described method.

In one of the example embodiment, the system and method for predicting effect of genomic variations on pre-mRNA splicing. In present embodiment, a recent experimentally determined 59,359 human branch sites (10 mers), identified based on exoribonuclease digestion and RNA-sequence is considered. The dataset offers a comprehensive dataset for training a high accuracy putative BPS prediction model (10). The present example, utilize this set of branch point sites, selecting only the sequences with 'A' at the branchpoint as the training set for the Position weight matrix (PWM) evaluator. This is because our goal is to create and evaluate a tool that can be used as part of a routine variant annotation scheme to provide high confidence annotations for further clinical interpretation. Parameters such as the distance of BPS from the 3' splice end (-15 to -50 nucleotides upstream) of the intron, making sure the BPS (branch point sequence) is part of the intronic region in all transcripts and setting a threshold on the basis of the top 25% scores in the PWM from the training set were chosen to increase the accuracy of the analysis approach. Comparisons to outcomes of other existing prediction tools like HSF (Human Splicing Finder), SVM (Support Vector Machine), BP finder, outputs of machine learning prediction tools, along with experimentally proven BPS mutations have been performed to demonstrate the accuracy of our proposed model.

The analysis method as described in accordance with the present embodiments, based on the PWM was successful in identifying the role of pathogenicity of 3 Clinvar annotated deleterious mutation cases (Table 1) in known branchpoints listed in the high confident branchpoint dataset is described below. The present analysis was successful in confirming the experimentally known cases of variants causing splicing aberrations due to activation of cryptic splice sites and branchpoint. The experiments were conducted for various known variants.

Example 1 - OTC: In an embodiment, a variant C>G in intron 9 was detected upon Clinvar based variant screening of Ornithine Carbamoyltransferase coding gene *(OTC)* as disrupting canonical splice acceptor site. Alternative splice acceptor site (MaxEnt: 8.30) was identified 25 bases downstream (in the exonic region) of the canonical splice acceptor junction. The canonical branchsite (score: 2.80) i.e. 29 bases upstream to the identified cryptic splice acceptor was deemed suitable. The inactivation of the canonical splice acceptor and activation of the cryptic acceptor site has been experimentally verified with the aid of PCR and the resulting aberration in splicing has been proven to cause an aberrant 50 amino C-terminal sequence in the protein resulting in hyperammoneamic crisis. The value corresponding to OTC are as shown in Table 1.

Example 2 *- MAN2B1*: In another embodiment, a T>C transition was found in intron 14 of Mannosidase Alpha Class 2B Member 1 gene (*MAN2B1*) disrupting the canonical splice acceptor site. Upon the loss of the canonical splice acceptor, a cryptic branch site is activated and also activation of a cryptic splice acceptor (MaxEnt: 4.78) 31nt downstream to the canonical 3' splice site occurs resulting in deletion of the first 31 nt of the exon 15, leading to a frame shift mutation causing pre-mature termination of the protein as a consequence of introduction of a stop codon (Table 1). With the aid of RT-PCR, the disruption of the canonical 3' splice acceptor site and the activation of the cryptic splice site leading to partial exon deletion has been confirmed. Overall, the analysis approach displayed the potential to unveil one of the causes behind deficiency of alpha-mannosidase.

**TABLE 1**

| Gene | Variant position | Sequence | Score |
|---|---|---|---|
| *OTC* | 38280273 | TTTCTTTGTTGTGTCAT[C>G]AG GCT | 7.73 > -1.02 |
| *MAN2B1* | 12763276 | GTGGACCCTTTCTGCCC[A>G] GCAC | 4.4 > -3.56 |

Experiments revealed some of the discovery cases. Herein, reason behind the splicing aberrations due to known pathogenic candidate variants was unveiled and such cases were categorized as discovery cases.

Example 3 - Alanine--Glyoxylate and Serine--Pyruvate Aminotransferase (AGXT): In an example embodiment, upon screening of the AGXT gene for variants, an A > G mutation was found in intron 5. As the variant is at the canonical splice acceptor site, it has been previously categorized as a splice site mutation, although the role of the variant and the specific effects on the splicing aberrations have not been defined. The canonical splice acceptor site of intron 5 was disrupted as a consequence of the variation (MaxEnt: 4.01>-3.94). Due to the disruption of the natural splice acceptor site, a cryptic splice acceptor site (MaxEnt: 5.01) 28 nucleotide downstream to the canonical splice acceptor site was activated. Further, upon screening for suitable branch sites for the cryptic splice acceptor, a potential branch site, i.e. 35 bases upstream to the cryptic splice acceptor site was found. Overall, on the basis of the proposed model it can be observed that due to the mutation, the original splice acceptor site gets disrupted and a cryptic splice acceptor, along with a cryptic branch point gets activated downstream to the canonical splice site and canonical branch site (Table 2). The resulting protein formed is 392 a.a long and loses 9 a.a i.e. an entire β-strand, in the core region as a result of the SNP. The deleted protein region forms a part of the active site and the homodimer interface of the protein and is essential for pyridoxal 5' phosphate binding. Therefore the deletion caused due to the SNP is highly deleterious as it causes protein dysfunctioning. A hypothesis can be drawn based on the occurrence of an alternative splice acceptor with a suitable branch site, leading to aberrant splicing. The pre-termination of the transcript due to the splicing disruption might be a cause to primary hyperoxaluria.

Example 4 - Myosin XVA *(MYO15A)*: In another embodiment, a deleterious variant G>A disrupting the canonical splice acceptor site was found upon screening of the intron 49 of *MYO15A* gene. As a result of the variant, a cryptic branch site (score: 1.92) was activated at the canonical splice acceptor junction. A cryptic splice acceptor site suitable for the cryptic branch site was activated 27 nt downstream (exonic region; MaxEnt: 7.13) to the canonical splice acceptor with the potential to cause partial exon 50 skipping or complete exon 50 skipping might occur as a result of using the stronger splice acceptor site of intron 50 (MaxEnt: 8.93) for splicing. The splicing aberration due to disruption of the canonical splice acceptor and the splicing consequences might be the cause behind non-syndromic genetic deafness. The resulting splicing aberrations do not lead to disruption of the frame of the protein but alter the protein region essential for peptide ligand binding with proline rich ligands like SH3 protein. SH3 domains in the protein are essential for intramolecular interactions leading to proper regulation of the enzymes and also in mediating multiprotein complex assemblies. Therefore, even though the frame of the protein is unaffected, essential active regions of the protein are altered leading to a truncated or non-functional protein. Overall, the analysis approach was successful in unveiling a hypothesis behind the effect of the intronic variant on splicing of intron 49 in *MYO15A* gene and the resulting pathogenicity.

Example 5 - Growth Hormone Receptor *(GHR):* In yet another example embodiment, a reinterpreted case, a splice acceptor variant (G>C) was identified upon screening of intron 8 of Growth Hormone Receptor. The variant being at the splice acceptor site (AG>AC) disrupted the canonical splice acceptor (MaxEnt: 5.55>-2.52) resulting in idiopathic short stature. Two different variant transcripts for *GHR* have been reported, one with complete skipping of exon 9 and the other with partial deletion of exon 9. The transcript with partial deletion of exon 9 was formed due to activation of a cryptic splice site downstream (24 nt) of the canonical splice acceptor. The occurrence of the splice variants has been reported but the cause behind their formation was not elucidated. The splice strength of the cryptic splice acceptor site (i.e. in the exonic region) is greater than the canonical splice acceptor site and the variant of interest disrupts the canonical splice acceptor site, leading to aberrant splicing, resulting in a non-functional protein due to premature termination of the protein. The variant has been associated with disruption of the canonical splice acceptor and exon 9 skipping indicating that the downstream cryptic splice acceptor was being unused for splicing. But based on the hypothesis drawn using the analysis model and the experimental evidence, GHR-(1-279) (splice variant), i.e. formed due to the activation of the cryptic splice acceptor site is as highly expressed as the canonical transcript, therefore upon disruption of the canonical splice acceptor, it is likely that the downstream cryptic splice acceptor would get activated instead of selecting the disrupted canonical splice acceptor site of the intron 10 leading to exon 9 skipping (Table 2). The protein product of GHR as a result of the variant loses 8 a.a from the part of the protein that forms part of the growth hormone binding protein (GHBP) after the cleavage from the *GHR.* Therefore deletion of such an essential region from the protein would lead to dysfunctioning of the protein and might be the cause behind the deleteriousness of the variant. Overall, the analysis approach was successful in reinterpreting the role of the deleterious variant (G>C) in GHR intron 8 splicing and pathogenicity causing growth hormone insensitivity.

**TABLE 2**

| Gene | Variant position | Sequence | Score |
|---|---|---|---|
| *AGXT* | 241813393 | AGCAAACCACCCATCTAC[A>C] GGCA | 4.01 > -3.94 |
| *MYO15A* | 18060469 | GACCCGAGCCTGGCCCATA[G> A]GCT | 3.14 > -5.61 |
| *GHR* | 42718153 | AAATTTATATGTTTTCAA[G>C] GAT | 5.55 > -2.52 |

In an embodiment, discoveries arising from predicted branch site variants were studied. Herein, experimentally known cases: The PWM based approach along with well-established splice site strength determination tool (MaxEnt) was tested on experimentally determined cases of branchpoint variants causing pathogenicity *(NTKR1, DYSF, TH).* The output of the analysis approach exactly reflected the experimental findings.

Example 6 - Neurotrophic receptor tyrosine kinase 1 (*NTRK1*): In an embodiment, based on the output of the predicted branchpoint variants, in the case of *NTRK1* (neurotrophic tyrosine kinase receptor family) gene, a putative branch site sequence, 31 bases upstream to the splice acceptor site, was screened with a deleterious variant T>A. The branch site score was drastically reduced after the mutation, 5.70>3.17 (Table 3) and a cryptic splice acceptor site was activated. The resulting spliced product after mutation comprised of insertion of an intronic (137 bp) segment attributed to the usage of the upstream cryptic splice acceptor site. Therefore the role of the T>A branch site mutation has been proven to be a major cause of congenital insensitivity to pain with anhidrosis (CIPA) and the analysis approach was successful in determining the same.

Example 7 - Dysferlin (*DYSF*): In yet another example embodiment, upon screening a deleterious mutation (A>G) in intron 31 of DYSF gene was identified. On the basis of the change in branch site scores it was revealed that the variant disrupts the branch site (Table 3). The deleterious mutation A>G has been experimentally verified to disrupt the branchpoint, leading to failure of lariat formation and skipping of exon 32 of dysferlin gene, resulting in recessively inherited limb-girdle muscular dystrophy type 2B (LGMD2B) and muscular dystrophies with distal presentations.

Example 8 - Tyrosine Hydroxylase (*TH*): In yet another example embodiment, the PWM based approach identified a putative branch site containing a deleterious variant T>A in intron 11 of *TH.* It has been proven that the deleterious variant leads to alternative splicing, via skipping of exon 12, resulting in absence of 32 amino acids in the final protein product, making it non-functional or usage of cryptic branch site resulting in aberrant splicing or via partial intron retention (36 nucleotides in the mRNA) resulting in incorporation of 12 additional amino acids, rendering the protein non-functional. The branch site scores for the predicted branch site reduced significantly as a result of the variant (Table 3).It has been proven that a branch site mutation (T>A ) in the gene of the enzyme tyrosine hydroxylase (*TH*) , two bases upstream of the branchpoint of intron 11 leads to aberrant protein product causing severe extrapyramidal movement disorder. The alternative splicing, leading to intron retention was also verified using the present method.

**TABLE 3**

| **Gene** | **BP position** | **Sequence** | **Score** |
|---|---|---|---|
| *NTRK1* | 156843392 | GCCC[T>A]GACCT | 5.701 > 3.174 |
| *DYSF* | 71817308 | CCACTC[A>G]CTC | 5.568 > Disrupted |
| *TH* | 2180717 | GGGC[T>A]GATGC | 4.206 > 1.679 |

In an embodiment, disruption of branchpoint causing splicing aberration resulting in exon skipping were validated.

Example 9 - glycogen phosphorylase, muscle associated (PYGM): In yet another example embodiment, from the predicted deleterious branchpoint variants in PYGM gene, a deleterious point mutation A>G was discovered in branch site sequence 'TCCCTGACAG' i.e. 26 bases upstream to the splice acceptor site of intron 3. This intronic mutation A>G has been experimentally proven to result in skipping of exon 4 leading to McArdle disease (17). Based on amplified PCR products from the natural and the mutated samples, retention of exon 4 was concluded and the variant was classified to be a splice acceptor site mutation but the role of the branch site was not addressed. Based on the proposed analysis approach and the scores obtained for the branch site strengths, the theory of exon 4 skipping is hypothesized to be due to the disruption of the canonical branchpoint (4.43 to null), which is 26 bases upstream to the canonical splice acceptor (Table 4). As the proximity of the variant to the canonical splice acceptor is 26 bases upstream and therefore is not likely to affect the splice site strength, the variant can be hypothesized to be a branch site mutation. Overall, the analysis approach was capable of determining and classifying an experimentally validated splice mutation as a branchpoint mutation.

Example 10 - Translocase Of Inner Mitochondrial Membrane 8A (*TIMM8A*): In yet another example embodiment, a deleterious variant in the putative branch site 'TTTGTGATTC' with the highest score 3.40 was identified 23 bases upstream to the splice acceptor site in the sole intron of Translocase Of Inner Mitochondrial Membrane 8 (*TIMM8A*) gene, *TIMM8A*/*DDP1* gene dysfunction leads to Mohr-Tranebjaerg syndrome or deafness /dystonia syndrome, there has been evidence of various missense and nonsense mutations in the coding regions of the exons of *TIMM8A.* There has been a recent finding of an intronic variant A>C causing X-linked dystonia deafness. The intronic variant in *TIMM8A* has been proven to cause protein dysfunction possibly due to splicing aberrations. The cause behind the splicing aberrations has not been discussed in terms of the branchpoint disruption. On the basis of the branchpoint scores obtained from the prediction tool, it was evident that the splicing aberration was due to branchpoint disruption (Table 3). Overall, the analysis was able to classify a proven intronic variant as a branchpoint mutation on the basis of the change in branch site scores (3.40 > null).

**TABLE 4**

| **Gene** | **BP position** | **Sequence** | **Score** |
|---|---|---|---|
| *PYGM* | 64525847 | TCCCTG[A>G]CAG | 4.430 > Disrupted |
| *TIMM8A* | 100601671 | TTTGTG[A>C]TTC | 3.401 > Disrupted |

In accordance with the present embodiments, the PWM based analysis approach is designed to screen for variants that are putative branch sites with 'A' as the branchpoint in any given sequence and determine the effect of a mutation in a branch site to the splicing of the intron. As observed in the aforementioned case studies the PWM of the present embodiments is able to identify putative branch sites in proximity to the intronic end. Also, the potential of the PWM is cross-checked with experimentally known branch sites identified by other tools and the outcome matched accurately. The cases studied discussed in detail revealed successful identification of known branchpoint mutations and also led to reinterpretation of certain cases indicating the cause behind speculated effects of splicing leading to a pathological condition.

The basis for the examples discussed above is the PWM matrix generated in accordance with the present embodiments. The PWM is created using a dataset of branch site 10 mer sequences containing adenosine as the branchpoint. The PWM was able to identify putative branch sites in proximity to the intronic end. The potential of the PWM was cross-checked with experimentally known branch sites identified by other tools and the outcome matched accurately. The analysis approach of the present method is focused on screening variants in branch sites with "A" as the branchpoint and studying the impact of the variant on splicing and the resulting pathogenicity. The examples, as observed, was successful in identification of known branchpoint mutations and also led to reinterpretation of certain cases indicating the cause behind speculated effects of splicing leading to a pathological condition. The input dataset upon variant screening shows a particular branchpoint variant in the *COL4A5* gene which was speculated to be a splice site variant but based on the scores obtained for the branch site before and after the mutation from the PWM created, indicated it to be a branchpoint mutation disrupting the branch site. The screening of putative branch site variants in the human genome, through the Clinvar.vcf successfully identified 20 cases with deleterious variants (pathogenic/likely pathogenic) as branch site mutations (TABLE 5) and 20 deleterious variants as splice site mutations (TABLE 6). An extra filter, that is, significant change in the branch site score/splice site acceptor score before and after the mutation was applied in order to pick drastically affected branchpoints/splice sites due to variation.

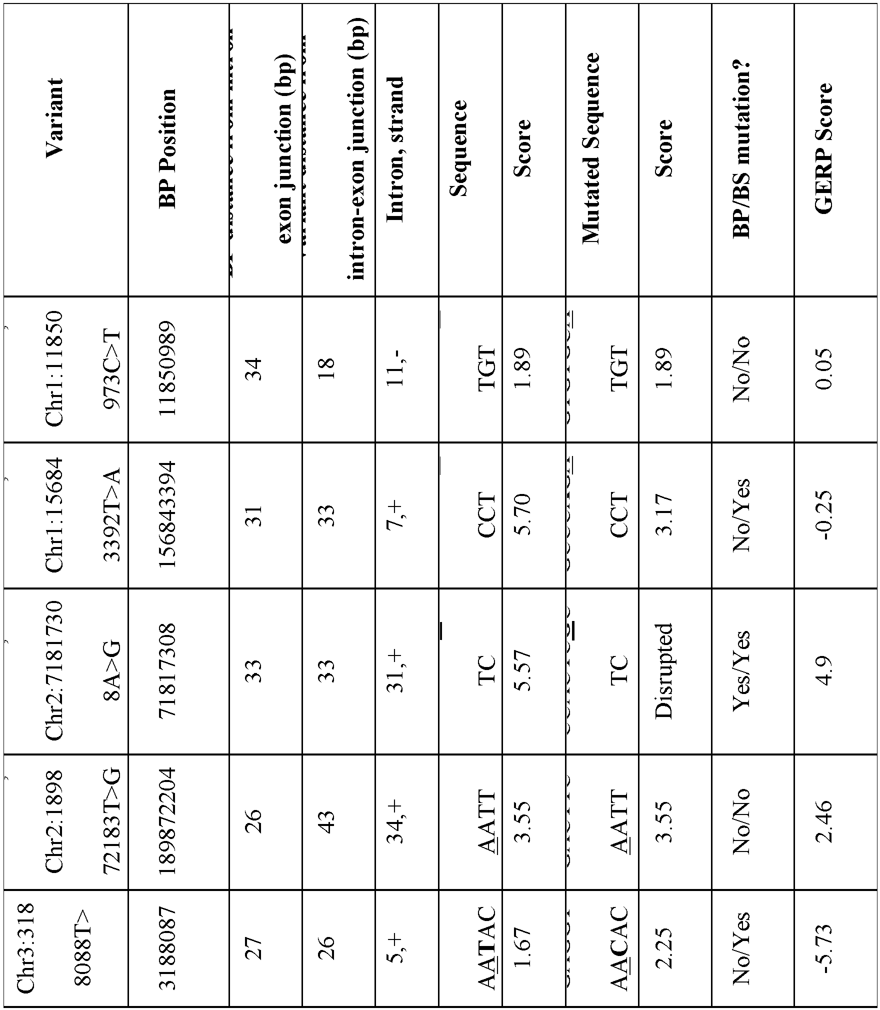

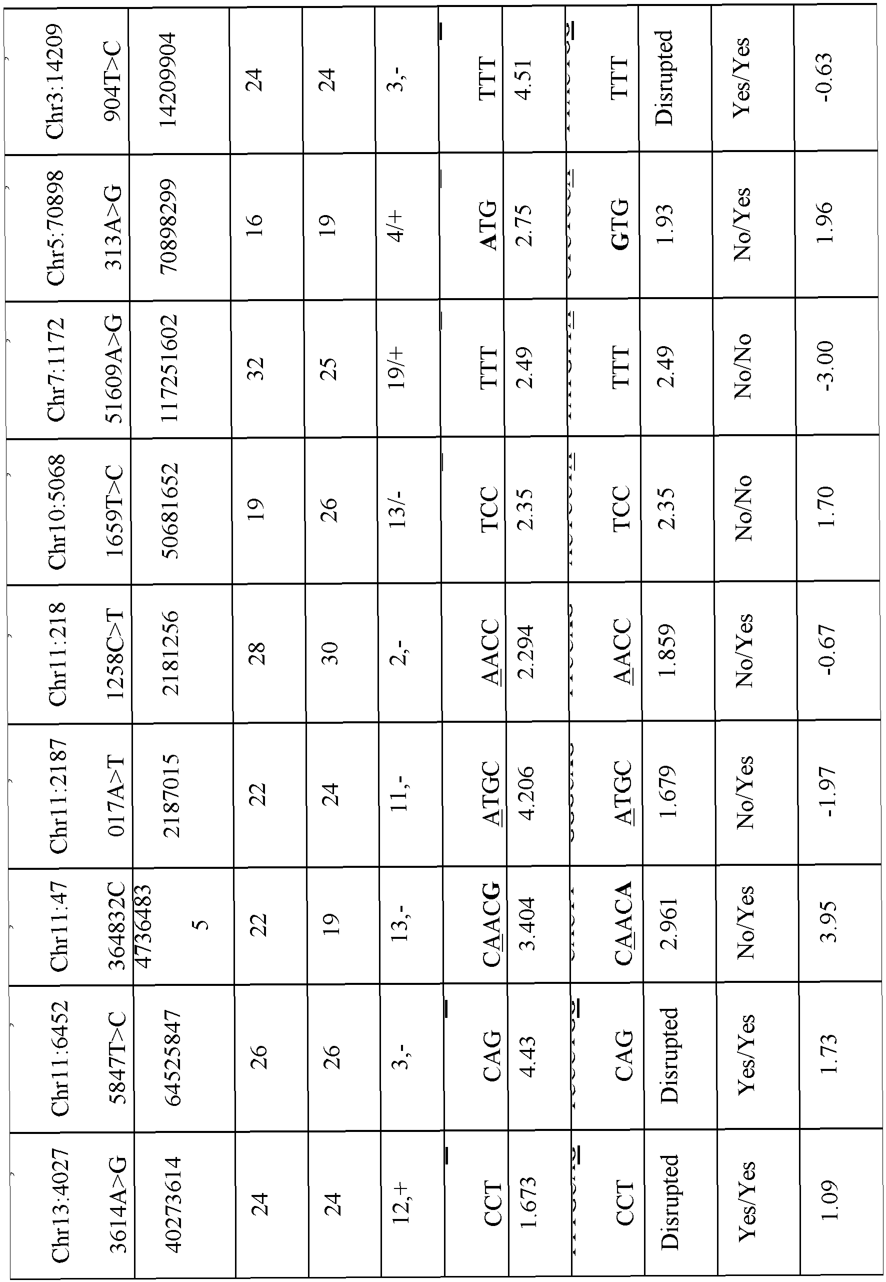

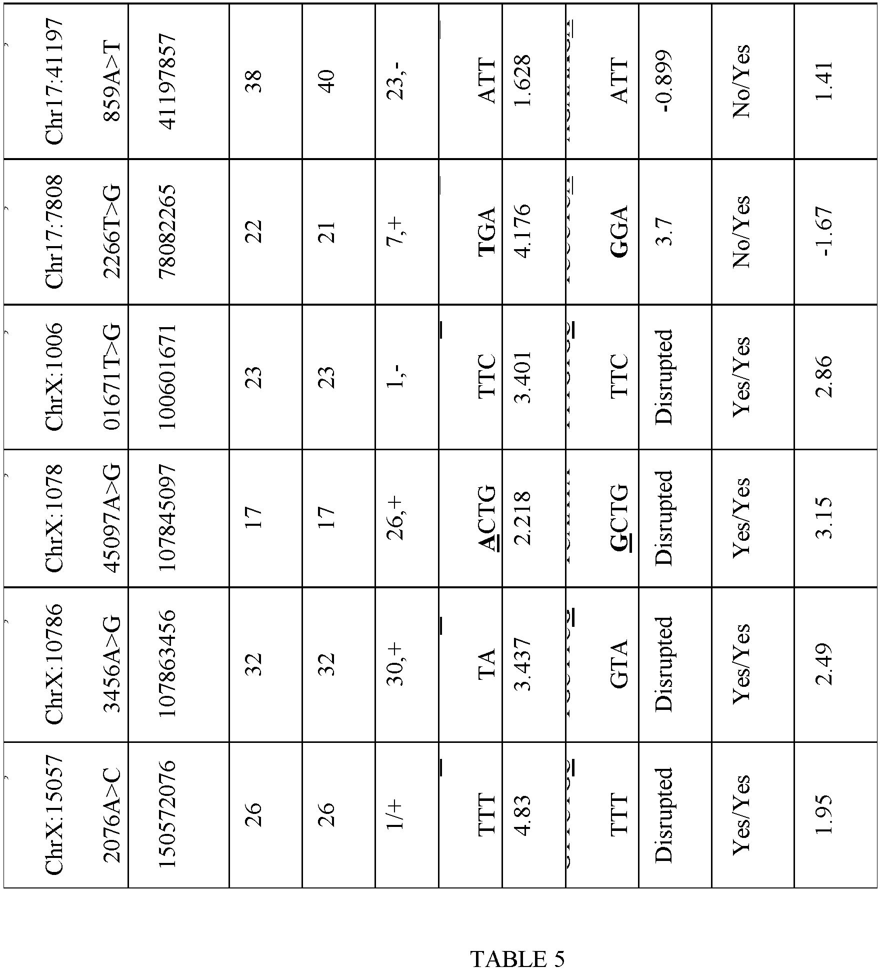

Out of the 20 potential branchpoint mutation cases, three cases of known i.e. experimentally verified branchpoint mutations and two discovery cases of mutations causing splicing aberrations in putative branchsites were successfully identified.

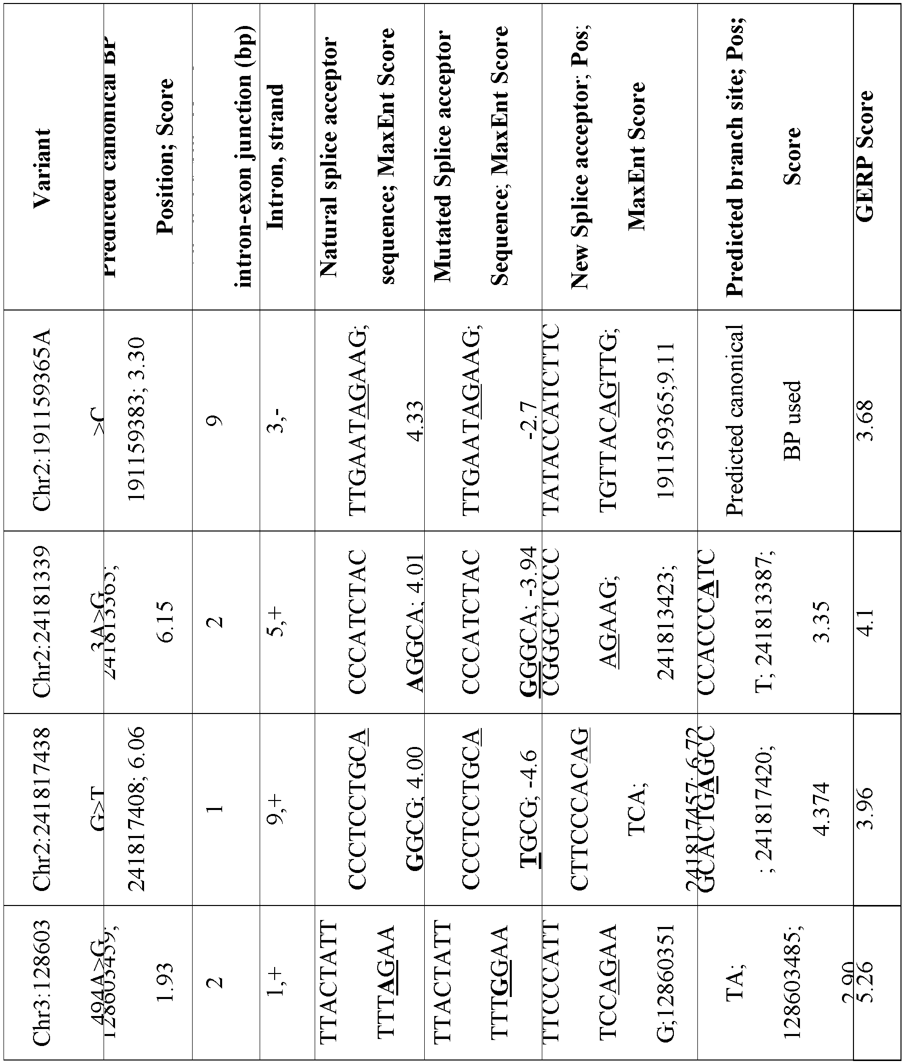

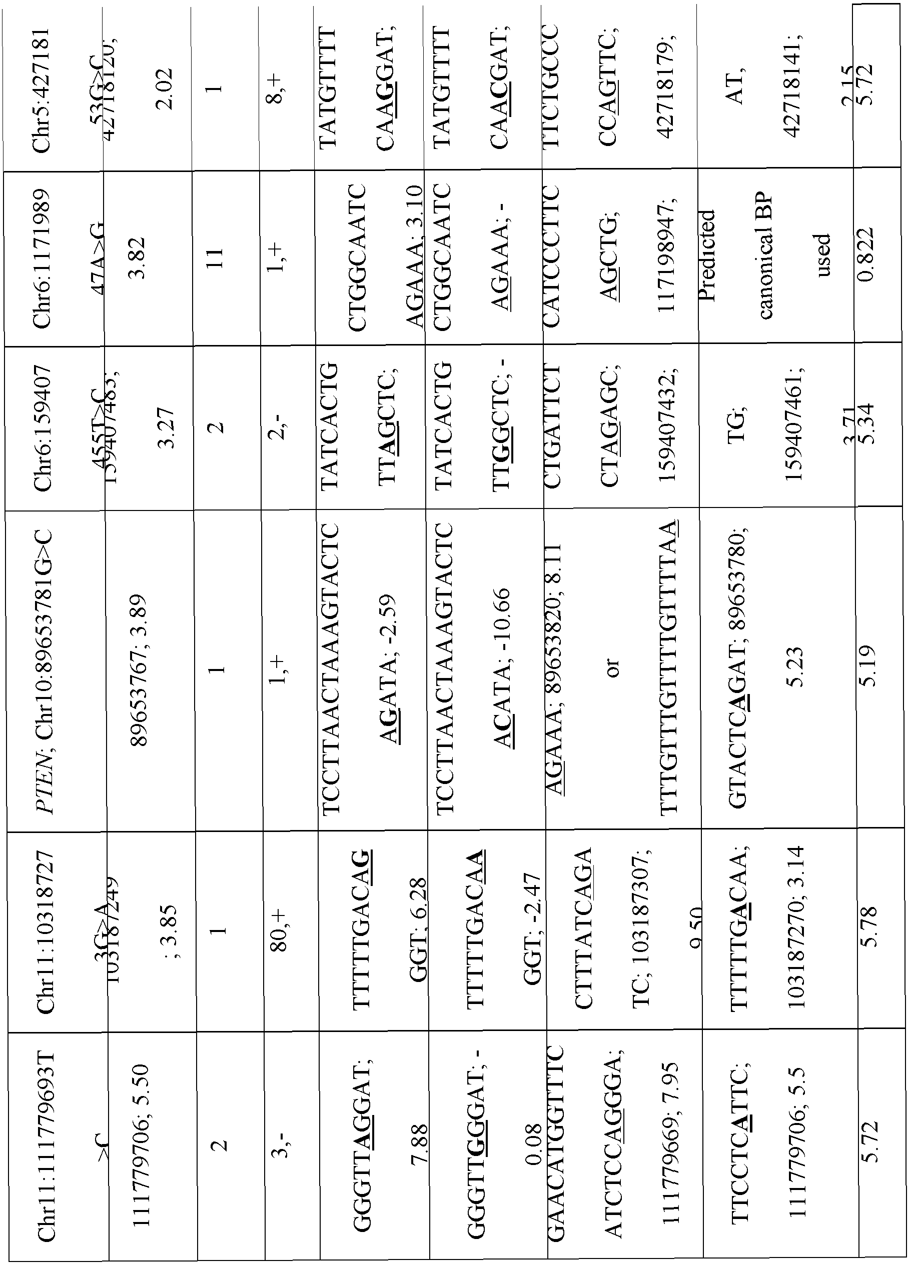

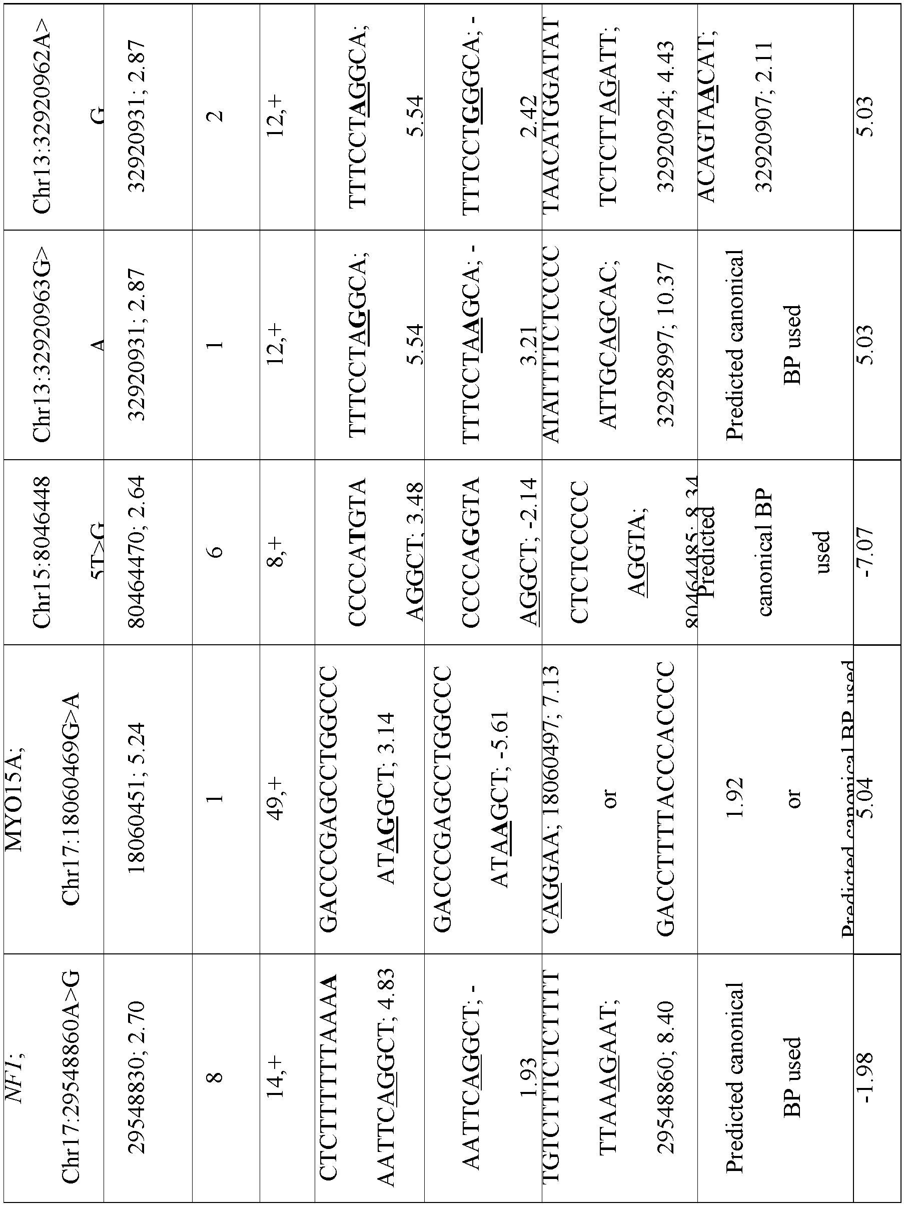

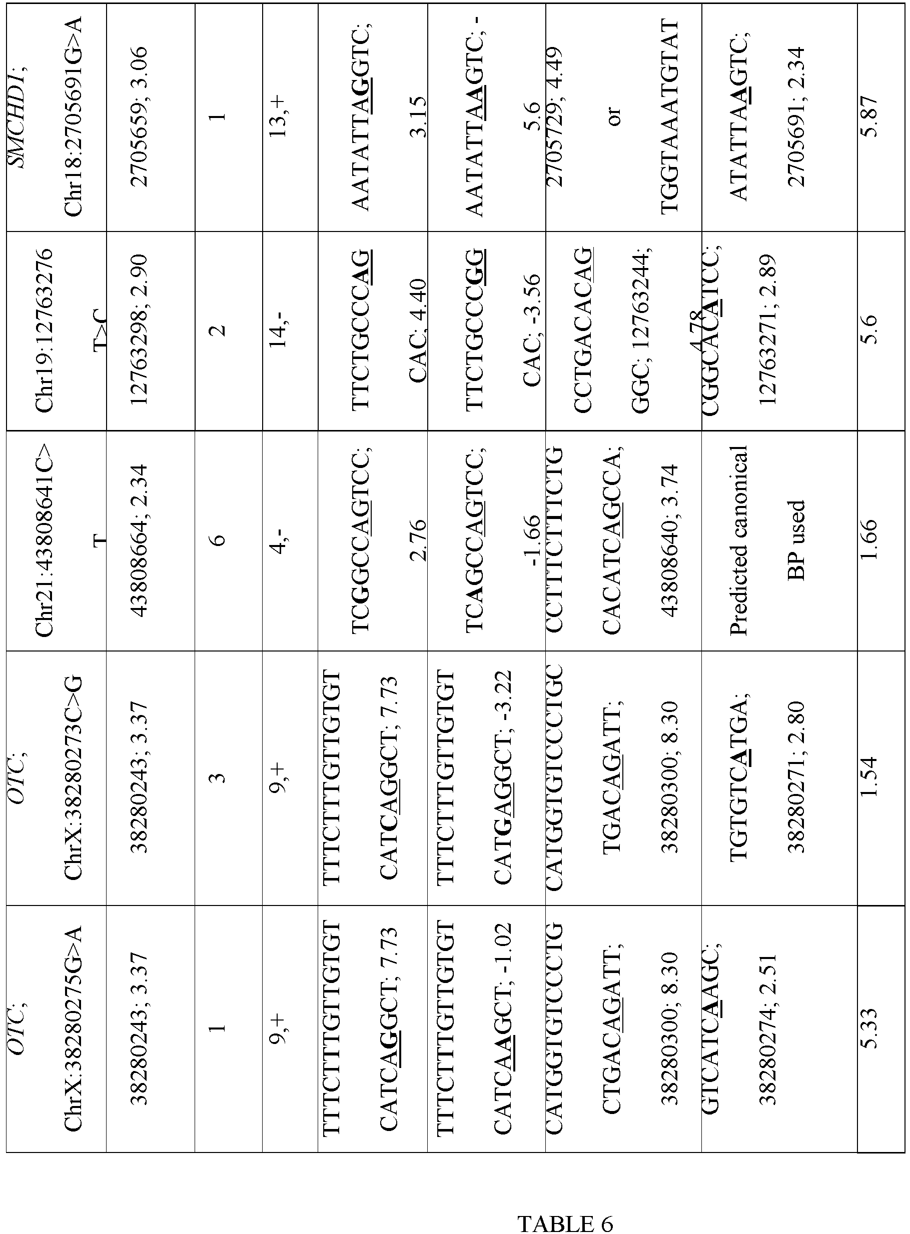

Alongside the variant screening within 15nt upstream to the intron/exon junction confirmed two experimentally proven cases Ornithine Carbamoyltransferase (OTC), Mannosidase Alpha Class 2B Member 1 (*MAN2B1*)), with variant disrupting canonical splice acceptor site leading to activation of cryptic splice acceptor site and cryptic branch site. The three known cases of branch site mutations and the two known cases of splice site mutations confirmed the potency of the analysis model in identifying potential branch sites in the introns (*NTRK1, DYSF, TH; OTC, MAN2B1*), while the two discovery cases of branch site mutations and splice site mutations (*PYGM, TIMM8A; AGXT, MYO15A*) confirms the potency of the analysis approach model in categorizing intronic variants as branchpoint or splice site variants based on the activation of a cryptic branchpoint or cryptic splice site. The analysis approach was also tested for the negative set i.e. the branchpoint variants that disrupt the branchpoint but cause no pathogenicity which shows that although the predicted branchpoint identified by the PWM tool was being disrupted, there were alternative branchpoints that were compensating for the disruption by enabling normal splicing of the intron. Therefore the analysis approach is successful in determining branchpoint variants and determining their pathogenicity based on the availability of alternative branchpoint which could rescue normal splicing.

As observed in the present examples, the present system and method proved successful in identifying variants that caused disruption of a branchpoint and led to creation of a new splice acceptor (Component of Oligomeric Golgi Complex 6 (*COG6*), Glucosidase Alpha, Acid (*GAA*)) at that site. It was also successful in identifying a putative splice acceptor site downstream to the canonical site upon creation of a new branchpoint at the canonical splice acceptor site as a result of the variation. In total, 40 variants with a potency to be a branch site or splice site mutation were identified and their role in causing splicing aberration was predicted with the aid of the designed tool. It was observed that few of the mutations did not affect the frame of the protein but were highly deleterious, for such cases, attributes like protein structure and function were checked. It was observed that for *AGXT,* Acyl-CoA Dehydrogenase Family Member 9 (*ACAD9*), *GHR, MYO15A* although the Single nucleotide polymorphisms (SNP) did not cause frame changes of the protein, it caused deletion of part of the active site of the protein affecting or ceasing the function leading to a disease condition. It was also noted that for certain cases like phosphatase and tensin homologue (*PTEN*), where exon skipping or partial exon deletion was predicted, the protein either is trucated or deletion of active site of the protein renders it non-functional. Overall, SNPs that affect the translational frame of the protein lead to pathogenicity most likely due to a truncated protein product and the SNPs that do not affect the translational frame of the protein lead to pathogenicity due to core regions of the protein being altered. The dataset obtained as a result of screening putative branchpoint mutations was compared against Human splicing factor dataset of identified putative branchpoints and was also compared against the identified branchpoint variants predicted results, which confirmed the PWM based analysis model is reliable for branchpoint prediction and for investigating splicing aberrations as a result of a branch site mutation or splice site mutation.

Therefore the PWM based approach is designed to screen for variants that are putative branch sites with 'A' as the branchpoint in any given sequence and determine the effect of a mutation in a branch site to the splicing of the intron.

The embodiments of the present system and method is capable of identifying branchpoint variants and along with other established tools that determine various aspects of splice site was successful in offering a more detailed biological explanation to the consequence of mutations. Also, the discovery cases is identified using the present embodiments hold strong potential in unveiling the cause behind known pathogenic conditions and provide basis for therapeutic developments. Prediction of putative branchpoint or splice site variants in an intron can lay the foundation for the identification of possible genotype-based therapies using exon-skipping techniques (TABLE 7).

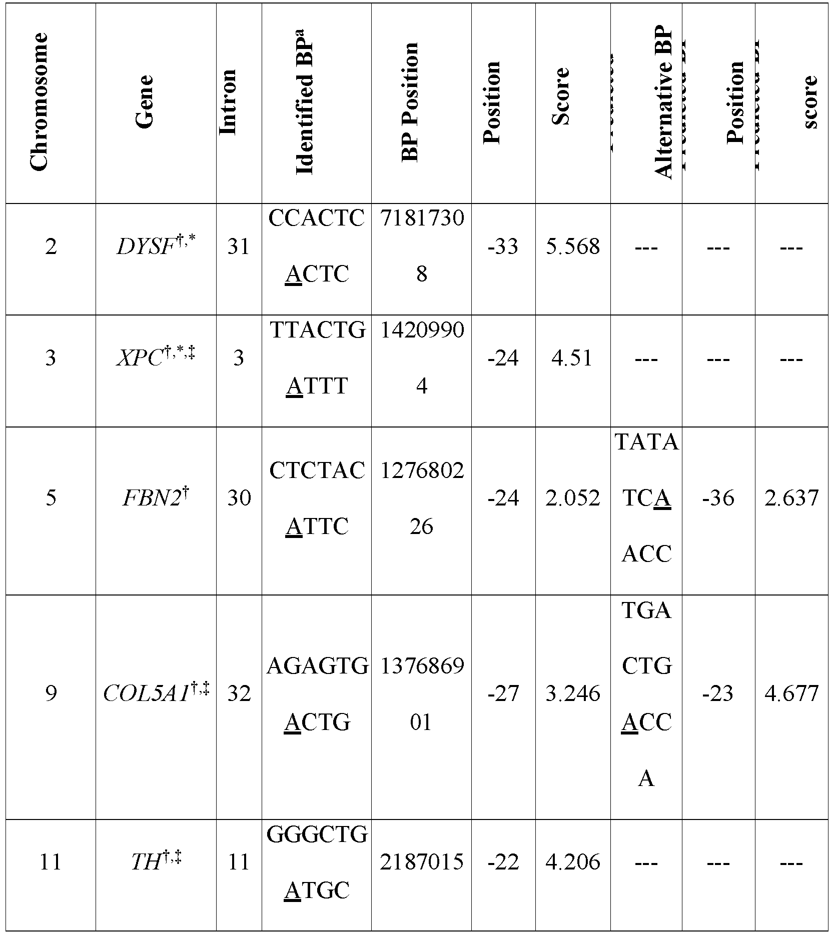

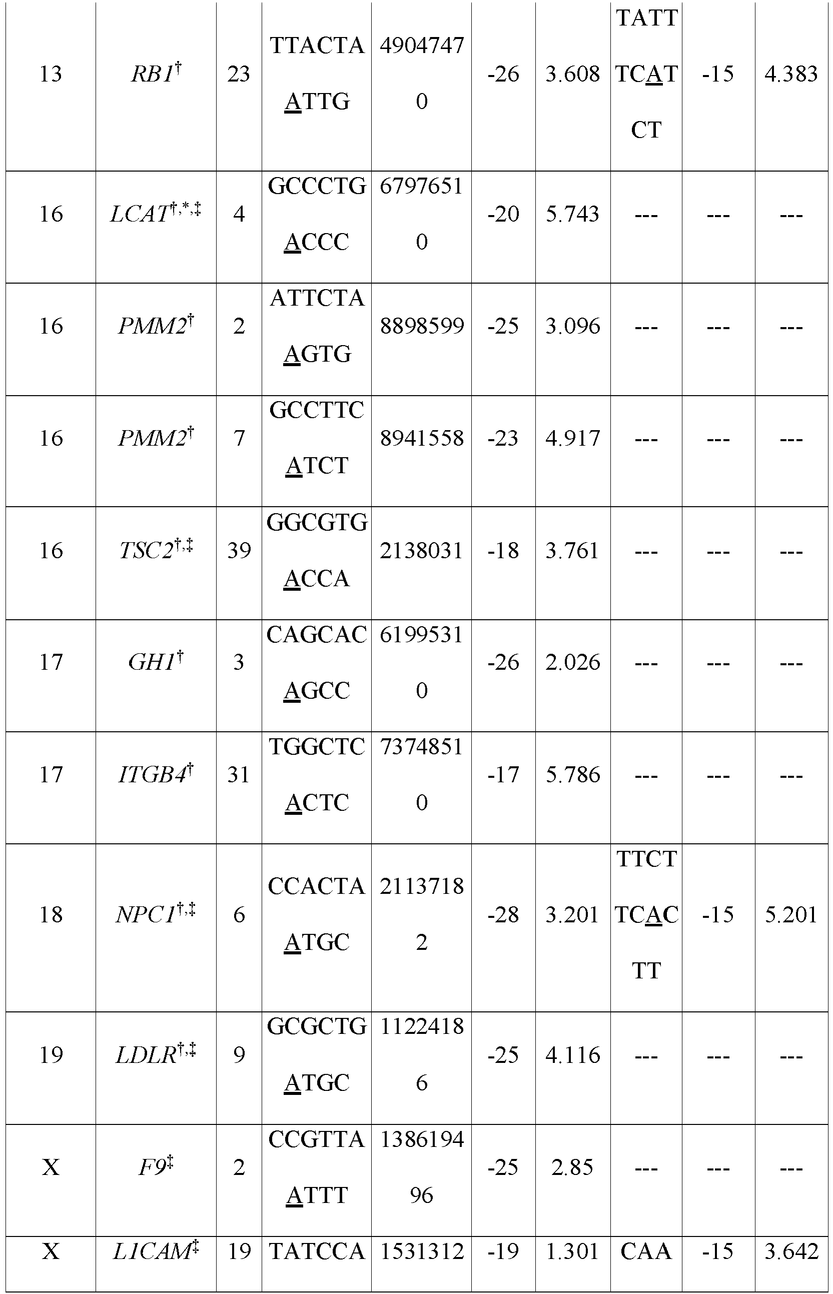

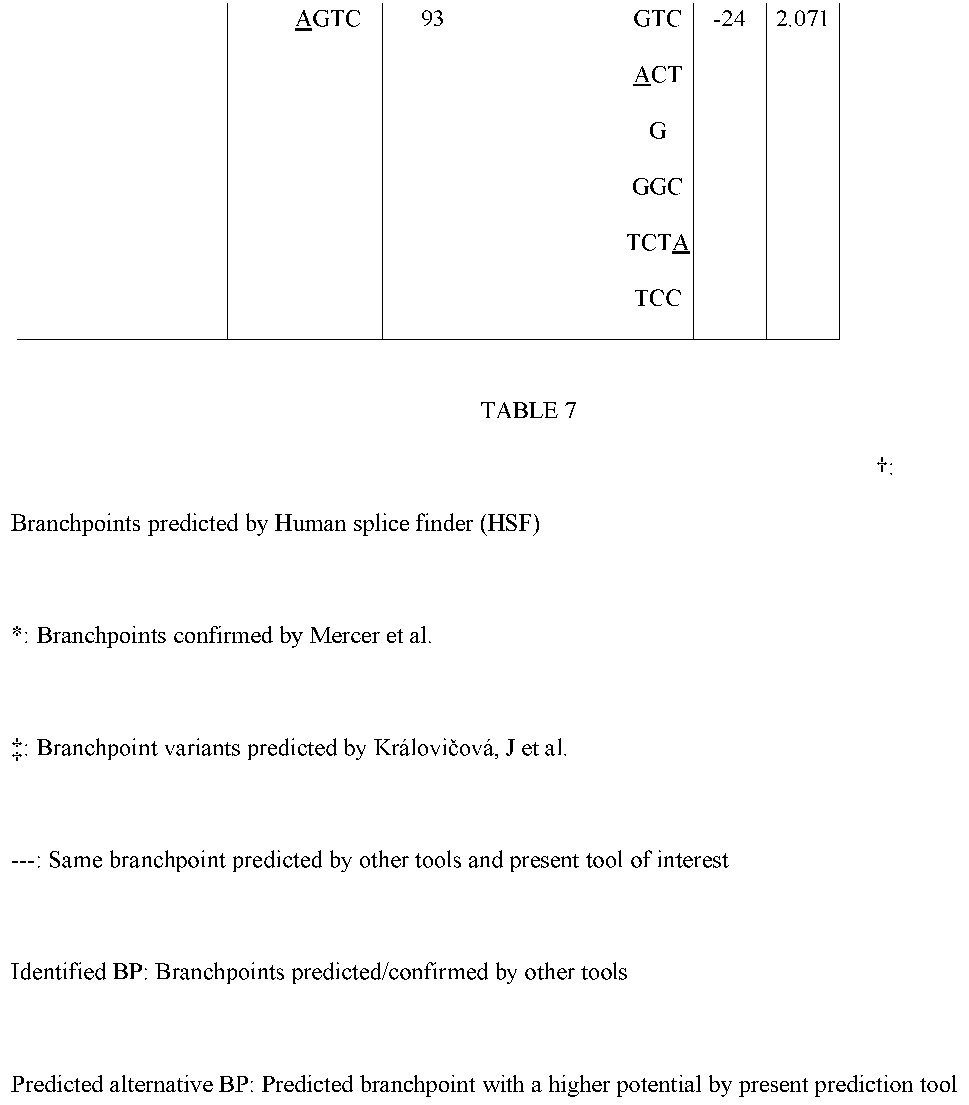

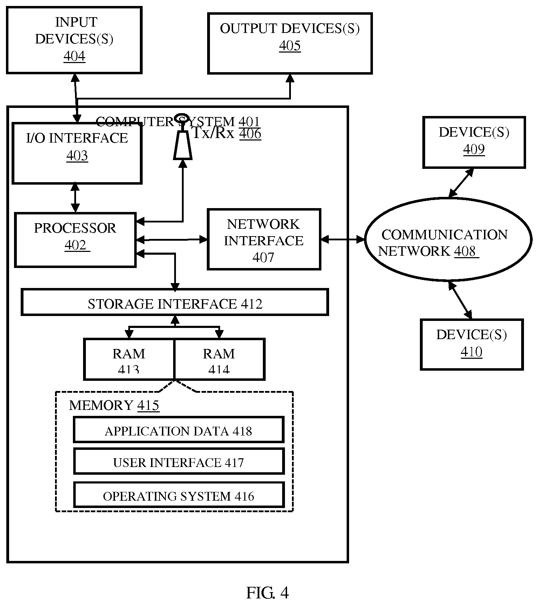

FIG. 4 is a block diagram of an exemplary computer system 401 for implementing embodiments consistent with the present disclosure. The computer system 401 may be implemented standalone or in combination of components of the system 102 (FIG. 1). Variations of computer system 401 may be used for implementing the devices included in this disclosure. Computer system 401 may comprise a central processing unit ("CPU" or "hardware processor") 402. The hardware processor 402 may comprise at least one data processor for executing program components for executing user- or system-generated requests. The processor may include specialized processing units such as integrated system (bus) controllers, memory management control units, floating point units, graphics processing units, digital signal processing units, etc. The processor may include a microprocessor, such as AMD Athlon^{™}, Duron^{™} or Opteron^{™}, ARM's application, embedded or secure processors, IBM PowerPCTM, Intel's Core, Itanium^{™}, Xeon^{™}, Celeron^{™} or other line of processors, etc. The processor 902 may be implemented using mainframe, distributed processor, multi-core, parallel, grid, or other architectures. Some embodiments may utilize embedded technologies like application specific integrated circuits (ASICs), digital signal processors (DSPs), Field Programmable Gate Arrays (FPGAs), etc.

Processor 402 may be disposed in communication with one or more input/output (I/O) devices via I/O interface 403. The I/O interface 403 may employ communication protocols/methods such as, without limitation, audio, analog, digital, monoaural, RCA, stereo, IEEE-1394, serial bus, universal serial bus (USB), infrared, PS/2, BNC, coaxial, component, composite, digital visual interface (DVI), high-definition multimedia interface (HDMI), RF antennas, S-Video, VGA, IEEE 402.11 a/b/g/n/x, Bluetooth, cellular (e.g., code-division multiple access (CDMA), high-speed packet access (HSPA+), global system for mobile communications (GSM), long-term evolution (LTE), WiMax, or the like), etc.

Using the I/O interface 403, the computer system 401 may communicate with one or more I/O devices. For example, the input device 404 may be an antenna, keyboard, mouse, joystick, (infrared) remote control, camera, card reader, fax machine, dongle, biometric reader, microphone, touch screen, touchpad, trackball, sensor (e.g., accelerometer, light sensor, GPS, gyroscope, proximity sensor, or the like), stylus, scanner, storage device, transceiver, video device/source, visors, etc.

Output device 405 may be a printer, fax machine, video display (e.g., cathode ray tube (CRT), liquid crystal display (LCD), light-emitting diode (LED), plasma, or the like), audio speaker, etc. In some embodiments, a transceiver 406 may be disposed in connection with the processor 402. The transceiver may facilitate various types of wireless transmission or reception. For example, the transceiver may include an antenna operatively connected to a transceiver chip (e.g., Texas Instruments WiLink WL1283, Broadcom BCM4750IUB8, Infineon Technologies X-Gold 618-PMB9800, or the like), providing IEEE 802.11a/b/g/n, Bluetooth, FM, global positioning system (GPS), 2G/3G HSDPA/HSUPA communications, etc.

In some embodiments, the processor 402 may be disposed in communication with a communication network 408 via a network interface 407. The network interface 407 may communicate with the communication network 408. The network interface may employ connection protocols including, without limitation, direct connect, Ethernet (e.g., twisted pair 10/100/1000 Base T), transmission control protocol/internet protocol (TCP/IP), token ring, IEEE 402.11a/b/g/n/x, etc. The communication network 408 may include, without limitation, a direct interconnection, local area network (LAN), wide area network (WAN), wireless network (e.g., using Wireless Application Protocol), the Internet, etc. Using the network interface 407 and the communication network 408, the computer system 401 may communicate with devices 409 and 410. These devices may include, without limitation, personal computer(s), server(s), fax machines, printers, scanners, various mobile devices such as cellular telephones, smartphones (e.g., Apple iPhone, Blackberry, Android-based phones, etc.), tablet computers, eBook readers (Amazon Kindle, Nook, etc.), laptop computers, notebooks, gaming consoles (Microsoft Xbox, Nintendo DS, Sony PlayStation, etc.), or the like. In some embodiments, the computer system 401 may itself embody one or more of these devices.

In some embodiments, the processor 402 may be disposed in communication with one or more memory devices (e.g., RAM 713, ROM 714, etc.) via a storage interface 412. The storage interface may connect to memory devices including, without limitation, memory drives, removable disc drives, etc., employing connection protocols such as serial advanced technology attachment (SATA), integrated drive electronics (IDE), IEEE-1394, universal serial bus (USB), fiber channel, small computer systems interface (SCSI), etc. The memory drives may further include a drum, magnetic disc drive, magneto-optical drive, optical drive, redundant array of independent discs (RAID), solid-state memory devices, solid-state drives, etc. Variations of memory devices may be used for implementing, for example, any databases utilized in this disclosure.

The memory devices may store a collection of program or database components, including, without limitation, an operating system 416, user interface application 417, user/application data 418 (e.g., any data variables or data records discussed in this disclosure), etc. The operating system 416 may facilitate resource management and operation of the computer system 401. Examples of operating systems include, without limitation, Apple Macintosh OS X, Unix, Unix-like system distributions (e.g., Berkeley Software Distribution (BSD), FreeBSD, NetBSD, OpenBSD, etc.), Linux distributions (e.g., Red Hat, Ubuntu, Kubuntu, etc.), IBM OS/2, Microsoft Windows (XP, Vista/7/8, etc.), Apple iOS, Google Android, Blackberry OS, or the like. User interface 417 may facilitate display, execution, interaction, manipulation, or operation of program components through textual or graphical facilities. For example, user interfaces may provide computer interaction interface elements on a display system operatively connected to the computer system 401, such as cursors, icons, check boxes, menus, scrollers, windows, widgets, etc. Graphical user interfaces (GUIs) may be employed, including, without limitation, Apple Macintosh operating systems' Aqua, IBM OS/2, Microsoft Windows (e.g., Aero, Metro, etc.), Unix X-Windows, web interface libraries (e.g., ActiveX, Java, Javascript, AJAX, HTML, Adobe Flash, etc.), or the like.

In some embodiments, computer system 401 may store user/application data 418, such as the data, variables, records, etc. as described in this disclosure. Such databases may be implemented as fault-tolerant, relational, scalable, secure databases such as Oracle or Sybase. Alternatively, such databases may be implemented using standardized data structures, such as an array, hash, linked list, structured text file (e.g., XML), table, or as object-oriented databases (e.g., using ObjectStore, Poet, Zope, etc.). Such databases may be consolidated or distributed, sometimes among the various computer systems discussed above in this disclosure. It is to be understood that the structure and operation of any computer or database component may be combined, consolidated, or distributed in any working combination.

Additionally, in some embodiments, the server, messaging and instructions transmitted or received may emanate from hardware, including operating system, and program code (i.e., application code) residing in a cloud implementation. Further, it should be noted that one or more of the systems and methods provided herein may be suitable for cloud-based implementation. For example, in some embodiments, some or all of the data used in the disclosed methods may be sourced from or stored on any cloud computing platform.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software modules located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various modules described herein may be implemented in other modules or combinations of other modules. For the purposes of this description, a computer-usable or computer readable medium can be any apparatus that can comprise, store, communicate, propagate, or transport the program for use by or in connection with the instruction execution system, apparatus, or device.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

## Claims

1. A processor-implemented method comprising:
receiving genomic position information of at least one candidate variant of gene transcripts and coordinates information of the gene transcripts, wherein each gene transcript is represented as a set of one or more non-overlapping intervals, where each non-overlapping interval is represented by four features including chromosome on which the gene transcript is present, a starting genomic coordinate of the non-overlapping interval, an ending genomic coordinate of the non-overlapping interval, and a strand on which the gene transcript is present, **characterized in that**:
classifying the at least one candidate variant into one of a splice acceptor site region and a branch site region based on the coordinates information of the gene transcripts and the genomic position information, wherein the at least one candidate variant is classified
as occuring in the splice acceptor site region having genomic coordinate between 15 nucleotides upstream to 3 nucleotides downstream of a natural intron-exon splice acceptor junction of the gene transcripts, and
as occuring in the branch site region having genomic coordinate between 50 nucleotides to 15 nucleotides upstream of a natural splice acceptor junction of the gene transcripts;
evaluating effect of the at least one candidate variant on pre-mRNA splicing, based on a classified region from the classification of the at least one candidate variant, wherein the evaluating the effect of the at least one candidate variant on the pre-mRNA splicing comprises:
identifying weakening of a natural splice acceptor site in the classified region, due to the at least one candidate variant, using MaxEnt score, wherein the MaxEnt score is splice site strength determination technique for calculating strength or weakening of the splice acceptor site, and wherein the MaxEnt score is assigned based on the effect of the at least one candidate variant on affected natural splice acceptor site region;
determining that a new splice acceptor site region is being created due to the weakened natural splice acceptor site; and
evaluating strength of an identified natural branchpoint in the classified region using Position Weight Matrix (PWM) evaluator in response to determining that the new splice acceptor site region being created, wherein the PWM evaluator is generated using experimentally determined human branch sites, wherein generating the PWM evaluator comprises:
filtering the determined human branch sites for 10mers having 'A' as a branchpoint;
aligning the filtered branch sites to calculate frequency of each of nucleotide at each position of the 10mers in the filtered branch sites;
normalizing the calculated frequency using a background frequency for each of the nucleotide at each position of the 10mers; and
constructing a (m*n) matrix using the normalized frequency to obtain the PWM, and wherein constructing the (m*n) matrix comprises converting each of the normalized frequencies to log odds values and constructing the (m*n) matrix into the PWM evaluator using the log odds values; and
wherein the step of evaluating effect of the at least one candidate variant on the splice acceptor site region for a new splice acceptor site being created comprises:
determining presence or absence of natural branchpoint in sequence range of 15 nucleotide to 50 nucleotide of the new splice acceptor site region being active during the pre-mRNA splicing, and
based on the determined presence or absence of the natural branchpoint,
evaluating strength of the natural branchpoint using the PWM evaluator and identifying the at least one candidate variant as disease causing based on the evaluated strength of the natural branchpoint; or
screening for an alternative branchpoint using the PWM evaluator and predicting the at least one candidate as a disease causing based on the evaluated strength of the alternative branchpoint,
wherein the method further comprises:
during the absence of the alternative branchpoint,
determining status of the natural splice acceptor site region, wherein the status comprising disruptive natural splice acceptor site region or non-disruptive natural splice acceptor site region; and
predicting the at least one candidate variant as disease causing or non-disease causing based on the determined status; and
predicting the disease causing ability of the at least one candidate variant based on the evaluated effect of the at least one candidate variant on the pre-mRNA splicing.

2. The processor implemented method of claim 1, wherein the generated PWM evaluator evaluates strength of branchpoint based on a threshold score, and wherein the threshold score is determined using a plurality of branch site scores obtained for branch sites with 'A' as the branchpoint.

3. The processor implemented method of claim 1, wherein the step of evaluating effect of the at least one candidate variant on the splice acceptor site region for no new splice acceptor site being created comprises:
determining effect of the at least one candidate variant on the natural branchpoint, and identifying level of strength of natural branch site using the PWM evaluator based on the determined effect;
screening for an alternative splice acceptor site region in sequence range having 50 nucleotide upstream and 50 nucleotide downstream of the at least one candidate variant and performing a comparison of strength of the alternative splice acceptor site region and weakened natural splice acceptor site region; and
determining presence of a new branchpoint being created and performing comparison of strength of the new branchpoint and the natural branchpoint,
wherein the method further comprises:
based on the identified level of the strength of natural branch site,
identifying the at least one candidate variant as a non-disease causing; or
identifying the at least one variant candidate as a disease causing or non-disease causing based on a screened alternative branchpoint in the sequence 50 nucleotide to 15 nucleotide upstream to the natural splice acceptor site region.
wherein the method further comprises:
based on the comparison of strength of alternative splice acceptor site region and weakened natural splice acceptor site:
predicting the at least one candidate variant as non- disease causing; or
determining presence of natural branchpoint in sequence range of 15 nucleotide to 50 nucleotide to the splice acceptor site region being active during the mRNA splicing and comparing strength of the natural branchpoint with the predefined threshold, wherein based on based on the determined presence and the comparison,
predicting the at least one candidate variant as disease causing or non-disease causing based on an alternative branchpoint screened in the sequence range of 50 nucleotides to 15 nucleotides upstream of the alternative splice acceptor site.

4. The processor implemented method of claim 3, further comprising based on the comparison of strength of the new branchpoint and the natural branchpoint,
predicting the at least one candidate variant as non- disease causing; or
determining presence of natural branchpoint in the range of 15 nucleotide to 50 nucleotide upstream to the splice acceptor site region being active during the pre-mRNA splicing and comparing strength of the natural branchpoint with the predefined threshold.

5. The processor implemented method of claim 3, based on the determined presence of natural branchpoint and comparison of strength of the natural branchpoint with the predefined threshold, predicting the at least one candidate variant as disease causing or non- disease causing.

6. The processor implemented method of claim 1, wherein the step of evaluating effect of the at least one candidate variant on the branch site for the new splice acceptor site being created comprises:
determining presence of an alternative branchpoint in sequence range having 50 nucleotides to 15 nucleotides upstream of the new splice acceptor site; and
predicting the at least one variant to be disease causing or non- disease causing based on the presence of the alternative branchpoint.

7. The processor implemented method of claim 1, wherein the step of evaluating effect of the at least one candidate variant on the branch site for no new splice acceptor site being created comprises:
screening for natural branchpoint in sequence range having 50 nucleotides to 15 nucleotides upstream of the natural splice acceptor site; and
determining level of strength of the branch site using the PWM evaluator, wherein determining the level of strength is due to the at least one candidate variant affecting the screened natural branchpoint,
wherein the method further comprises:
based on the determined level of strength of the branch site,
predicting the at least one candidate variant as disease causing; or
predicting the at least one candidate variant as disease causing or non-disease causing based on an alternative branchpoint screened in sequence range of 50 nucleotides to 15 nucleotides upstream of the natural splice acceptor site region.

8. A system (102) comprising:
a memory storing instructions;
one or more hardware processors coupled to the memory, wherein the one or more hardware processors are configured by the instructions to:
receive genomic position information of at least one candidate variant of gene transcripts and coordinates information of the gene transcripts, wherein each transcript is represented as a set of one or more non-overlapping intervals, where each non-overlapping interval is represented by four features that include chromosome on which the transcript is present, a starting genomic coordinate of the non-overlapping interval, an ending genomic coordinate of the non-overlapping interval, and a strand on which the transcript is present, **characterized in that**:
classify the at least one candidate variant into one of a splice acceptor site region and a branch site region based on the coordinates information of the gene transcripts and the genomic position information of at least one candidate variant, wherein the at least one candidate variant is classified
as occuring in the splice acceptor site region having genomic coordinate between 15 nucleotides upstream to 3 nucleotides downstream of a natural intron-exon splice acceptor junction of the gene transcripts, and
as occuring in the branch site region having genomic coordinate between 50 nucleotides to 15 nucleotides upstream of a natural splice acceptor junction of the gene transcripts;
evaluate effect of the at least one candidate variant on pre-mRNA splicing, based on a classified region from the classification of the at least one candidate variant, wherein the evaluating the effect of the at least one candidate variant on the pre-mRNA splicing comprises:
identifying weakening of a natural splice acceptor site in the classified region, due to the at least one candidate variant, using MaxEnt score, wherein the MaxEnt score is splice site strength determination technique for calculating strength or weakening of the splice acceptor site, and wherein the MaxEnt score is assigned based on the effect of the at least one candidate variant on affected natural splice acceptor site region;
determining that a new splice acceptor site region is being created due to the weakened natural splice acceptor site; and
evaluating strength of an identified natural branchpoint in the classified region using PWM evaluator in response to determining that the new splice acceptor site region being created, wherein the PWM evaluator is generated using experimentally determined human branch sites, wherein generating the PWM evaluator comprises:
filtering the determined human branch sites for 10mers having 'A' as a branchpoint;
aligning the filtered branch sites to calculate frequency of each of nucleotide at each position of the 10mers in the filtered branch sites;
normalizing the calculated frequency using a background frequency for each of the nucleotide at each position of the 10mers; and
constructing a (m*n) matrix using the normalized frequency to obtain the PWM, and wherein constructing the (m*n) matrix comprises converting each of the normalized frequencies to log odds values and constructing the (m*n) matrix into the PWM evaluator using the log odds values; and
wherein the step of evaluating effect of the at least one candidate variant on the splice acceptor site region for a new splice acceptor site being created comprises:
determining presence or absence of natural branchpoint in sequence range of 15 nucleotide to 50 nucleotide of the new splice acceptor site region being active during the pre-mRNA splicing, and
based on the determined presence or absence of the natural branchpoint,
evaluating strength of the natural branchpoint using the PWM evaluator and identifying the at least one candidate variant as disease causing based on the evaluated strength of the natural branchpoint; or
screening for an alternative branchpoint using the PWM evaluator and predicting the at least one candidate as a disease causing based on the evaluated strength of the alternative branchpoint,
wherein the method further comprises:
during the absence of the alternative branchpoint,
determining status of the natural splice acceptor site region, wherein the status comprising disruptive natural splice acceptor site region or non-disruptive natural splice acceptor site region; and
predicting the at least one candidate variant as disease causing or non-disease causing based on the determined status; and; and
predict the disease causing ability of the at least one candidate variant based on the evaluated effect of the at least one candidate variant on pre-mRNA splicing.

9. The system of claim 8, wherein evaluating the effect of the at least one candidate variant on the splice acceptor site region for no new splice acceptor site being created comprises:
determining effect of the at least one candidate variant on the natural branchpoint, and identifying level of strength of natural branch site using the PWM evaluator based on the determined effect;
screening for an alternative splice acceptor site region in sequence range having 50 nucleotide upstream and 50 nucleotide downstream of the at least one candidate variant and performing a comparison of strength of the alternative splice acceptor site region and weakened natural splice acceptor site region; and
determining presence of a new branchpoint being created and performing comparison of strength of the new branchpoint and the natural branchpoint.
wherein the one or more hardware processors are further configured by the instructions based on the identified level of the strength of natural branch site to:
identify the at least one candidate variant as a non- disease causing; or
identify the at least one variant candidate as a disease causing or non- disease causing based on a screened alternative branchpoint in the sequence 50 nucleotide to 15 nucleotide upstream to the natural splice acceptor site region.
wherein the one or more hardware processors are further configured by the instructions based on the comparison of strength of alternative splice acceptor site region and weakened natural splice acceptor site to:
predict the at least one candidate variant as non- disease causing; or
determine presence of natural branchpoint in sequence range of 15 nucleotide to 50 nucleotide to the splice acceptor site region being active during the mRNA splicing and comparing strength of the natural branchpoint with the predefined threshold.
wherein the one or more hardware processors are further configured by the instructions based on the comparison of strength of the new branchpoint and the natural branchpoint to:
predict the at least one candidate variant as non- disease causing; or
determine presence of natural branchpoint in the range of 15 nucleotide to 50 nucleotide upstream to the splice acceptor site region being active during the mRNA splicing and comparing strength of the natural branchpoint with the predefined threshold,
further comprising based on the determined presence and the comparison, predicting the at least one candidate variant as disease causing or non- disease causing based on an alternative branchpoint screened in the sequence range of 50 nucleotides to 15 nucleotides upstream of the alternative splice acceptor site,
wherein the one or more hardware processors are further configured based on the determined presence of natural branchpoint and comparison of strength of the natural branchpoint with the predefined threshold to:
predict the at least one candidate variant as disease causing or non- disease causing.

10. The system of claim 8, wherein evaluating the effect of the at least one candidate variant on the branch site for the new splice acceptor site being created comprises:
determining presence of an alternative branchpoint in sequence range having 50 nucleotides to 15 nucleotides upstream of the new splice acceptor site; and
predicting the at least one variant to be disease causing or non- disease causing based on the presence of the alternative branchpoint.
wherein the one or more hardware processors are further configured by the instructions to:
evaluate the effect of the at least one candidate variant on the branch site for no new splice acceptor site being created comprises:
screen for natural branchpoint in sequence range having 50 nucleotides to 15 nucleotides upstream of the natural splice acceptor site; and
determine level of strength of the branch site using the PWM evaluator , wherein determining the level of strength is due to the at least one candidate variant affecting the screened natural branchpoint,
wherein the one or more hardware processors are further configured based on the determined level of strength of the branch site to:
predict the at least one candidate variant as disease causing; or
predict the at least one candidate variant as disease causing or non- disease causing based on an alternative branchpoint screened in sequence range of 50 nucleotides to 15 nucleotides upstream of the natural splice acceptor site region.

## Patentansprüche

1. Prozessorimplementiertes Verfahren, umfassend:
Empfangen von genomischen Positionsinformationen von mindestens einer Kandidatenvariante von Gentranskripten und Koordinateninformationen der Gentranskripte, wobei jedes Gentranskript als ein Satz von einem oder mehreren nicht überlappenden Intervallen dargestellt wird, wobei jedes nicht überlappende Intervall durch vier Merkmale dargestellt wird, einschließlich eines Chromosoms, auf dem das Gentranskript vorhanden ist, einer genomischen Startkoordinate des nicht überlappenden Intervalls, einer genomischen Endkoordinate des nicht überlappenden Intervalls und eines Strangs, auf dem das Gentranskript vorhanden ist, **dadurch gekennzeichnet, dass**:
Klassifizieren der mindestens einen Kandidatenvariante in eine von einer Spleißakzeptorstellenregion und einer Verzweigungsstellenregion basierend auf den Koordinateninformationen der Gentranskripte und den genomischen Positionsinformationen, wobei die mindestens eine Kandidatenvariante klassifiziert wird
als in der Spleißakzeptorstellenregion mit einer genomischen Koordinate zwischen 15 Nukleotiden stromaufwärts bis 3 Nukleotiden stromabwärts einer natürlichen Intron-Exon-Spleißakzeptorverbindung der Gentranskripte vorkommend, und
als in der Verzweigungsstellenregion mit einer genomischen Koordinate zwischen 50 Nukleotiden bis 15 Nukleotiden stromaufwärts einer natürlichen Spleißakzeptorverbindung der Gentranskripte vorkommend;
Bewerten der Wirkung der mindestens einen Kandidatenvariante auf das Prä-mRNA-Spleißen basierend auf einer klassifizierten Region aus der Klassifizierung der mindestens einen Kandidatenvariante, wobei das Bewerten der Wirkung der mindestens einen Kandidatenvariante auf das Prä-mRNA-Spleißen umfasst:
Identifizieren einer Schwächung einer natürlichen Spleißakzeptorstelle in der klassifizierten Region aufgrund der mindestens einen Kandidatenvariante unter Verwendung eines MaxEnt-Scores, wobei der MaxEnt-Score eine Spleißstellenstärkebestimmungstechnik zum Berechnen der Stärke oder Schwächung der Spleißakzeptorstelle ist, und wobei der MaxEnt-Score basierend auf der Wirkung der mindestens einen Kandidatenvariante auf die betroffene natürliche Spleißakzeptorstellenregion zugewiesen wird;
Bestimmen, dass eine neue Spleißakzeptorstellenregion aufgrund der geschwächten natürlichen Spleißakzeptorstelle erzeugt wird; und
Bewerten der Stärke eines identifizierten natürlichen Verzweigungspunkts in der klassifizierten Region unter Verwendung eines Positionsgewichtsmatrix(PWM)-Bewerters als Reaktion auf das Bestimmen, dass die neue Spleißakzeptorstellenregion erzeugt wird, wobei der PWM-Bewerter unter Verwendung experimentell bestimmter menschlicher Verzweigungsstellen erzeugt wird, wobei das Erzeugen des PWM-Bewerters umfasst:
Filtern der bestimmten menschlichen Verzweigungsstellen für 10mere mit "A" als Verzweigungspunkt;
Ausrichten der gefilterten Verzweigungsstellen, um die Häufigkeit jedes Nukleotids an jeder Position der 10mere in den gefilterten Verzweigungsstellen zu berechnen;
Normalisieren der berechneten Häufigkeit unter Verwendung einer Hintergrundhäufigkeit für jedes der Nukleotide an jeder Position der 10mere; und
Konstruieren einer (m*n)-Matrix unter Verwendung der normalisierten Häufigkeit, um die PWM zu erhalten, und wobei das Konstruieren der (m*n)-Matrix das Umwandeln jeder der normalisierten Häufigkeiten in log-Odds-Werte und das Konstruieren der (m*n)-Matrix in den PWM-Bewerter unter Verwendung der log-Odds-Werte umfasst; und
wobei der Schritt des Bewertens der Wirkung der mindestens einen Kandidatenvariante auf die Spleißakzeptorstellenregion für eine neue Spleißakzeptorstelle, die erzeugt wird, umfasst:
Bestimmen des Vorhandenseins oder Nichtvorhandenseins eines natürlichen Verzweigungspunkts im Sequenzbereich von 15 Nukleotiden bis 50 Nukleotiden der neuen Spleißakzeptorstellenregion, die während des Prä-mRNA-Spleißens aktiv ist, und
basierend auf dem bestimmten Vorhandensein oder Nichtvorhandensein des natürlichen Verzweigungspunkts,
Bewerten der Stärke des natürlichen Verzweigungspunkts unter Verwendung des PWM-Bewerters und Identifizieren der mindestens einen Kandidatenvariante als krankheitsverursachend basierend auf der bewerteten Stärke des natürlichen Verzweigungspunkts; oder
Screenen nach einem alternativen Verzweigungspunkt unter Verwendung des PWM-Bewerters und Vorhersagen des mindestens einen Kandidaten als krankheitsverursachend basierend auf der bewerteten Stärke des alternativen Verzweigungspunkts,
wobei das Verfahren ferner umfasst:
während des Nichtvorhandenseins des alternativen Verzweigungspunkts, Bestimmen des Status der natürlichen Spleißakzeptorstellenregion, wobei der Status eine störende natürliche Spleißakzeptorstellenregion oder eine nicht störende natürliche Spleißakzeptorstellenregion umfasst; und
Vorhersagen der mindestens einen Kandidatenvariante als krankheitsverursachend oder nicht krankheitsverursachend basierend auf dem bestimmten Status; und
Vorhersagen der krankheitsverursachenden Fähigkeit der mindestens einen Kandidatenvariante basierend auf der bewerteten Wirkung der mindestens einen Kandidatenvariante auf das Prä-mRNA-Spleißen.

2. Prozessorimplementiertes Verfahren nach Anspruch 1, wobei der erzeugte PWM-Bewerter die Stärke des Verzweigungspunkts basierend auf einer Schwellenbewertung bewertet, und wobei die Schwellenbewertung unter Verwendung einer Mehrzahl von Verzweigungsstellenbewertungen bestimmt wird, die für Verzweigungsstellen mit "A" als Verzweigungspunkt erhalten werden.

3. Prozessorimplementiertes Verfahren nach Anspruch 1, wobei der Schritt des Bewertens der Wirkung der mindestens einen Kandidatenvariante auf die Spleißakzeptorstellenregion für keine neue Spleißakzeptorstelle, die erzeugt wird, umfasst:
Bestimmen der Wirkung der mindestens einen Kandidatenvariante auf den natürlichen Verzweigungspunkt und Identifizieren des Stärkeniveaus der natürlichen Verzweigungsstelle unter Verwendung des PWM-Bewerters basierend auf der bestimmten Wirkung;
Screenen nach einer alternativen Spleißakzeptorstellenregion im Sequenzbereich mit 50 Nukleotiden stromaufwärts und 50 Nukleotiden stromabwärts der mindestens einen Kandidatenvariante und Durchführen eines Vergleichs der Stärke der alternativen Spleißakzeptorstellenregion und der geschwächten natürlichen Spleißakzeptorstellenregion; und
Bestimmen des Vorhandenseins eines neuen Verzweigungspunkts, der erzeugt wird, und Durchführen eines Vergleichs der Stärke des neuen Verzweigungspunkts und des natürlichen Verzweigungspunkts,
wobei das Verfahren ferner umfasst:
basierend auf dem identifizierten Stärkeniveau der natürlichen Verzweigungsstelle,
Identifizieren der mindestens einen Kandidatenvariante als nicht krankheitsverursachend; oder
Identifizieren der mindestens einen Kandidatenvariante als krankheitsverursachend oder nicht krankheitsverursachend basierend auf einem gescreenten alternativen Verzweigungspunkt in der Sequenz 50 Nukleotide bis 15 Nukleotide stromaufwärts der natürlichen Spleißakzeptorstellenregion.
wobei das Verfahren ferner umfasst:
basierend auf dem Vergleich der Stärke der alternativen Spleißakzeptorstellenregion und der geschwächten natürlichen Spleißakzeptorstelle:
Vorhersagen der mindestens einen Kandidatenvariante als nicht krankheitsverursachend; oder
Bestimmen des Vorhandenseins eines natürlichen Verzweigungspunkts im Sequenzbereich von 15 Nukleotiden bis 50 Nukleotiden der Spleißakzeptorstellenregion, die während des mRNA-Spleißens aktiv ist, und Vergleichen der Stärke des natürlichen Verzweigungspunkts mit dem vordefinierten Schwellenwert, wobei basierend auf dem bestimmten Vorhandensein und dem Vergleich,
Vorhersagen der mindestens einen Kandidatenvariante als krankheitsverursachend oder nicht krankheitsverursachend basierend auf einem gescreenten alternativen Verzweigungspunkt im Sequenzbereich von 50 Nukleotiden bis 15 Nukleotiden stromaufwärts der alternativen Spleißakzeptorstelle.

4. Prozessorimplementiertes Verfahren nach Anspruch 3, ferner umfassend, basierend auf dem Vergleich der Stärke des neuen Verzweigungspunkts und des natürlichen Verzweigungspunkts,
Vorhersagen der mindestens einen Kandidatenvariante als nicht krankheitsverursachend; oder
Bestimmen des Vorhandenseins eines natürlichen Verzweigungspunkts im Bereich von 15 Nukleotiden bis 50 Nukleotiden stromaufwärts der Spleißakzeptorstellenregion, die während des Prä-mRNA-Spleißens aktiv ist, und Vergleichen der Stärke des natürlichen Verzweigungspunkts mit dem vordefinierten Schwellenwert.

5. Prozessorimplementiertes Verfahren nach Anspruch 3, basierend auf dem bestimmten Vorhandensein eines natürlichen Verzweigungspunkts und dem Vergleich der Stärke des natürlichen Verzweigungspunkts mit dem vordefinierten Schwellenwert, Vorhersagen der mindestens einen Kandidatenvariante als krankheitsverursachend oder nicht krankheitsverursachend.

6. Prozessorimplementiertes Verfahren nach Anspruch 1, wobei der Schritt des Bewertens der Wirkung der mindestens einen Kandidatenvariante auf die Verzweigungsstelle für die neue Spleißakzeptorstelle, die erzeugt wird, umfasst:
Bestimmen des Vorhandenseins eines alternativen Verzweigungspunkts im Sequenzbereich mit 50 Nukleotiden bis 15 Nukleotiden stromaufwärts der neuen Spleißakzeptorstelle; und
Vorhersagen der mindestens einen Variante als krankheitsverursachend oder nicht krankheitsverursachend basierend auf dem Vorhandensein des alternativen Verzweigungspunkts.

7. Prozessorimplementiertes Verfahren nach Anspruch 1, wobei der Schritt des Bewertens der Wirkung der mindestens einen Kandidatenvariante auf die Verzweigungsstelle für keine neue Spleißakzeptorstelle, die erzeugt wird, umfasst:
Screenen nach einem natürlichen Verzweigungspunkt im Sequenzbereich mit 50 Nukleotiden bis 15 Nukleotiden stromaufwärts der natürlichen Spleißakzeptorstelle; und
Bestimmen des Stärkeniveaus der Verzweigungsstelle unter Verwendung des PWM-Bewerters, wobei das Bestimmen des Stärkeniveaus darauf zurückzuführen ist, dass die mindestens eine Kandidatenvariante den gescreenten natürlichen Verzweigungspunkt beeinflusst,
wobei das Verfahren ferner umfasst:
basierend auf dem bestimmten Stärkeniveau der Verzweigungsstelle,
Vorhersagen der mindestens einen Kandidatenvariante als krankheitsverursachend; oder
Vorhersagen der mindestens einen Kandidatenvariante als krankheitsverursachend oder nicht krankheitsverursachend basierend auf einem gescreenten alternativen Verzweigungspunkt im Sequenzbereich von 50 Nukleotiden bis 15 Nukleotiden stromaufwärts der natürlichen Spleißakzeptorstellenregion.

8. System (102), umfassend:
einen Speicher, der Anweisungen speichert;
einen oder mehrere Hardwareprozessoren, die mit dem Speicher gekoppelt sind, wobei der eine oder die mehreren Hardwareprozessoren durch die Anweisungen konfiguriert sind zum:
Empfangen von genomischen Positionsinformationen von mindestens einer Kandidatenvariante von Gentranskripten und Koordinateninformationen der Gentranskripte, wobei jedes Transkript als ein Satz von einem oder mehreren nicht überlappenden Intervallen dargestellt wird, wobei jedes nicht überlappende Intervall durch vier Merkmale dargestellt wird, einschließlich eines Chromosoms, auf dem das Transkript vorhanden ist, einer genomischen Startkoordinate des nicht überlappenden Intervalls, einer genomischen Endkoordinate des nicht überlappenden Intervalls und eines Strangs, auf dem das Transkript vorhanden ist, **dadurch gekennzeichnet, dass**:
Klassifizieren der mindestens einen Kandidatenvariante in eine von einer Spleißakzeptorstellenregion und einer Verzweigungsstellenregion basierend auf den Koordinateninformationen der Gentranskripte und den genomischen Positionsinformationen von mindestens einer Kandidatenvariante, wobei die mindestens eine Kandidatenvariante klassifiziert wird
als in der Spleißakzeptorstellenregion mit einer genomischen Koordinate zwischen 15 Nukleotiden stromaufwärts bis 3 Nukleotiden stromabwärts einer natürlichen Intron-Exon-Spleißakzeptorverbindung der Gentranskripte vorkommend, und
als in der Verzweigungsstellenregion mit einer genomischen Koordinate zwischen 50 Nukleotiden bis 15 Nukleotiden stromaufwärts einer natürlichen Spleißakzeptorverbindung der Gentranskripte vorkommend;
Bewerten der Wirkung der mindestens einen Kandidatenvariante auf das Prä-mRNA-Spleißen basierend auf einer klassifizierten Region aus der Klassifizierung der mindestens einen Kandidatenvariante, wobei das Bewerten der Wirkung der mindestens einen Kandidatenvariante auf das Prä-mRNA-Spleißen umfasst:
Identifizieren einer Schwächung einer natürlichen Spleißakzeptorstelle in der klassifizierten Region aufgrund der mindestens einen Kandidatenvariante unter Verwendung eines MaxEnt-Scores, wobei der MaxEnt-Score eine Spleißstellenstärkebestimmungstechnik zum Berechnen der Stärke oder Schwächung der Spleißakzeptorstelle ist, und wobei der MaxEnt-Score basierend auf der Wirkung der mindestens einen Kandidatenvariante auf die betroffene natürliche Spleißakzeptorstellenregion zugewiesen wird;
Bestimmen, dass eine neue Spleißakzeptorstellenregion aufgrund der geschwächten natürlichen Spleißakzeptorstelle erzeugt wird; und
Bewerten der Stärke eines identifizierten natürlichen Verzweigungspunkts in der klassifizierten Region unter Verwendung eines PWM-Bewerters als Reaktion auf das Bestimmen, dass die neue Spleißakzeptorstellenregion erzeugt wird, wobei der PWM-Bewerter unter Verwendung experimentell bestimmter menschlicher Verzweigungsstellen erzeugt wird, wobei das Erzeugen des PWM-Bewerters umfasst:
Filtern der bestimmten menschlichen Verzweigungsstellen für 10mere mit "A" als Verzweigungspunkt;
Ausrichten der gefilterten Verzweigungsstellen, um die Häufigkeit jedes Nukleotids an jeder Position der 10mere in den gefilterten Verzweigungsstellen zu berechnen;
Normalisieren der berechneten Häufigkeit unter Verwendung einer Hintergrundhäufigkeit für jedes der Nukleotide an jeder Position der 10mere; und
Konstruieren einer (m*n)-Matrix unter Verwendung der normalisierten Häufigkeit, um die PWM zu erhalten, und wobei das Konstruieren der (m*n)-Matrix das Umwandeln jeder der normalisierten Häufigkeiten in log-Odds-Werte und das Konstruieren der (m*n)-Matrix in den PWM-Bewerter unter Verwendung der log-Odds-Werte umfasst; und
wobei der Schritt des Bewertens der Wirkung der mindestens einen Kandidatenvariante auf die Spleißakzeptorstellenregion für eine neue Spleißakzeptorstelle, die erzeugt wird, umfasst:
Bestimmen des Vorhandenseins oder Nichtvorhandenseins eines natürlichen Verzweigungspunkts im Sequenzbereich von 15 Nukleotiden bis 50 Nukleotiden der neuen Spleißakzeptorstellenregion, die während des Prä-mRNA-Spleißens aktiv ist, und
basierend auf dem bestimmten Vorhandensein oder Nichtvorhandensein des natürlichen Verzweigungspunkts,
Bewerten der Stärke des natürlichen Verzweigungspunkts unter Verwendung des PWM-Bewerters und Identifizieren der mindestens einen Kandidatenvariante als krankheitsverursachend basierend auf der bewerteten Stärke des natürlichen Verzweigungspunkts; oder
Screenen nach einem alternativen Verzweigungspunkt unter Verwendung des PWM-Bewerters und Vorhersagen des mindestens einen Kandidaten als krankheitsverursachend basierend auf der bewerteten Stärke des alternativen Verzweigungspunkts,
wobei das Verfahren ferner umfasst:
während des Nichtvorhandenseins des alternativen Verzweigungspunkts, Bestimmen des Status der natürlichen Spleißakzeptorstellenregion, wobei der Status eine störende natürliche Spleißakzeptorstellenregion oder eine nicht störende natürliche Spleißakzeptorstellenregion umfasst; und
Vorhersagen der mindestens einen Kandidatenvariante als krankheitsverursachend oder nicht krankheitsverursachend basierend auf dem bestimmten Status; und
Vorhersagen der krankheitsverursachenden Fähigkeit der mindestens einen Kandidatenvariante basierend auf der bewerteten Wirkung der mindestens einen Kandidatenvariante auf das Prä-mRNA-Spleißen.

9. System nach Anspruch 8, wobei das Bewerten der Wirkung der mindestens einen Kandidatenvariante auf die Spleißakzeptorstellenregion für keine neue Spleißakzeptorstelle, die erzeugt wird, umfasst:
Bestimmen der Wirkung der mindestens einen Kandidatenvariante auf den natürlichen Verzweigungspunkt und Identifizieren des Stärkeniveaus der natürlichen Verzweigungsstelle unter Verwendung des PWM-Bewerters basierend auf der bestimmten Wirkung;
Screenen nach einer alternativen Spleißakzeptorstellenregion im Sequenzbereich mit 50 Nukleotiden stromaufwärts und 50 Nukleotiden stromabwärts der mindestens einen Kandidatenvariante und Durchführen eines Vergleichs der Stärke der alternativen Spleißakzeptorstellenregion und der geschwächten natürlichen Spleißakzeptorstellenregion; und
Bestimmen des Vorhandenseins eines neuen Verzweigungspunkts, der erzeugt wird, und Durchführen eines Vergleichs der Stärke des neuen Verzweigungspunkts und des natürlichen Verzweigungspunkts.
wobei der eine oder die mehreren Hardwareprozessoren ferner durch die Anweisungen basierend auf dem identifizierten Stärkeniveau der natürlichen Verzweigungsstelle konfiguriert sind zum:
Identifizieren der mindestens einen Kandidatenvariante als nicht krankheitsverursachend; oder
Identifizieren der mindestens einen Kandidatenvariante als krankheitsverursachend oder nicht krankheitsverursachend basierend auf einem gescreenten alternativen Verzweigungspunkt in der Sequenz 50 Nukleotide bis 15 Nukleotide stromaufwärts der natürlichen Spleißakzeptorstellenregion.
wobei der eine oder die mehreren Hardwareprozessoren ferner durch die Anweisungen basierend auf dem Vergleich der Stärke der alternativen Spleißakzeptorstellenregion und der geschwächten natürlichen Spleißakzeptorstelle konfiguriert sind zum:
Vorhersagen der mindestens einen Kandidatenvariante als nicht krankheitsverursachend; oder
Bestimmen des Vorhandenseins eines natürlichen Verzweigungspunkts im Sequenzbereich von 15 Nukleotiden bis 50 Nukleotiden der Spleißakzeptorstellenregion, die während des mRNA-Spleißens aktiv ist, und Vergleichen der Stärke des natürlichen Verzweigungspunkts mit dem vordefinierten Schwellenwert.
wobei der eine oder die mehreren Hardwareprozessoren ferner durch die Anweisungen basierend auf dem Vergleich der Stärke des neuen Verzweigungspunkts und des natürlichen Verzweigungspunkts konfiguriert sind zum:
Vorhersagen der mindestens einen Kandidatenvariante als nicht krankheitsverursachend; oder
Bestimmen des Vorhandenseins eines natürlichen Verzweigungspunkts im Bereich von 15 Nukleotiden bis 50 Nukleotiden stromaufwärts der Spleißakzeptorstellenregion, die während des mRNA-Spleißens aktiv ist, und Vergleichen der Stärke des natürlichen Verzweigungspunkts mit dem vordefinierten Schwellenwert,
ferner umfassend basierend auf dem bestimmten Vorhandensein und dem Vergleich, Vorhersagen der mindestens einen Kandidatenvariante als krankheitsverursachend oder nicht krankheitsverursachend basierend auf einem gescreenten alternativen Verzweigungspunkt im Sequenzbereich von 50 Nukleotiden bis 15 Nukleotiden stromaufwärts der alternativen Spleißakzeptorstelle,
wobei der eine oder die mehreren Hardwareprozessoren ferner basierend auf dem bestimmten Vorhandensein eines natürlichen Verzweigungspunkts und dem Vergleich der Stärke des natürlichen Verzweigungspunkts mit dem vordefinierten Schwellenwert konfiguriert sind zum:
Vorhersagen der mindestens einen Kandidatenvariante als krankheitsverursachend oder nicht krankheitsverursachend.

10. System nach Anspruch 8, wobei das Bewerten der Wirkung der mindestens einen Kandidatenvariante auf die Verzweigungsstelle für die neue Spleißakzeptorstelle, die erzeugt wird, umfasst:
Bestimmen des Vorhandenseins eines alternativen Verzweigungspunkts im Sequenzbereich mit 50 Nukleotiden bis 15 Nukleotiden stromaufwärts der neuen Spleißakzeptorstelle; und
Vorhersagen der mindestens einen Variante als krankheitsverursachend oder nicht krankheitsverursachend basierend auf dem Vorhandensein des alternativen Verzweigungspunkts.
wobei der eine oder die mehreren Hardwareprozessoren ferner durch die Anweisungen konfiguriert sind zum:
Bewerten der Wirkung der mindestens einen Kandidatenvariante auf die Verzweigungsstelle für keine neue Spleißakzeptorstelle, die erzeugt wird, umfasst:
Screenen nach einem natürlichen Verzweigungspunkt im Sequenzbereich mit 50 Nukleotiden bis 15 Nukleotiden stromaufwärts der natürlichen Spleißakzeptorstelle; und
Bestimmen des Stärkeniveaus der Verzweigungsstelle unter Verwendung des PWM-Bewerters, wobei das Bestimmen des Stärkeniveaus darauf zurückzuführen ist, dass die mindestens eine Kandidatenvariante den gescreenten natürlichen Verzweigungspunkt beeinflusst,
wobei der eine oder die mehreren Hardwareprozessoren ferner basierend auf dem bestimmten Stärkeniveau der Verzweigungsstelle konfiguriert sind zum:
Vorhersagen der mindestens einen Kandidatenvariante als krankheitsverursachend; oder
Vorhersagen der mindestens einen Kandidatenvariante als krankheitsverursachend oder nicht krankheitsverursachend basierend auf einem gescreenten alternativen Verzweigungspunkt im Sequenzbereich von 50 Nukleotiden bis 15 Nukleotiden stromaufwärts der natürlichen Spleißakzeptorstellenregion.

## Revendications

1. Procédé mis en oeuvre par processeur comprenant :
la réception d'informations de position génomique d'au moins un variant candidat de transcrits de gènes et d'informations de coordonnées des transcrits de gènes, dans lequel chaque transcrit de gène est représenté comme un ensemble d'un ou plusieurs intervalles non chevauchants, où chaque intervalle non chevauchant est représenté par quatre caractéristiques incluant un chromosome sur lequel le transcrit de gène est présent, une coordonnée génomique de départ de l'intervalle non chevauchant, une coordonnée génomique de fin de l'intervalle non chevauchant, et un brin sur lequel le transcrit de gène est présent, **caractérisé en ce que** :
la classification de l'au moins un variant candidat dans l'une d'une région de site accepteur d'épissage et d'une région de site de ramification sur la base des informations de coordonnées des transcrits de gènes et des informations de position génomique, dans lequel l'au moins un variant candidat est classé
comme se produisant dans la région de site accepteur d'épissage ayant une coordonnée génomique entre 15 nucléotides en amont et 3 nucléotides en aval d'une jonction accepteur d'épissage intron-exon naturel des transcrits de gènes, et
comme se produisant dans la région de site de ramification ayant une coordonnée génomique entre 50 nucléotides et 15 nucléotides en amont d'une jonction accepteur d'épissage naturel des transcrits de gènes ;
l'évaluation de l'effet de l'au moins un variant candidat sur l'épissage de pré-ARNm, sur la base d'une région classée à partir de la classification de l'au moins un variant candidat, dans lequel l'évaluation de l'effet de l'au moins un variant candidat sur l'épissage de pré-ARNm comprend :
l'identification d'un affaiblissement d'un site accepteur d'épissage naturel dans la région classée, dû à l'au moins un variant candidat, en utilisant un score MaxEnt, dans lequel le score MaxEnt est une technique de détermination de force de site d'épissage pour calculer une force ou un affaiblissement du site accepteur d'épissage, et dans lequel le score MaxEnt est attribué sur la base de l'effet de l'au moins un variant candidat sur la région de site accepteur d'épissage naturel affectée
la détermination qu'une nouvelle région de site accepteur d'épissage est en cours de création en raison du site accepteur d'épissage naturel affaibli ; et
l'évaluation d'une force d'un point de ramification naturel identifié dans la région classée en utilisant un évaluateur de matrice de pondération de position (PWM) en réponse à la détermination que la nouvelle région de site accepteur d'épissage est en cours de création, dans lequel l'évaluateur PWM est généré en utilisant des sites de ramification humains déterminés expérimentalement, dans lequel la génération de l'évaluateur PWM comprend :
le filtrage des sites de ramification humains déterminés pour des décamères ayant « A » en tant que point de ramification ;
l'alignement des sites de ramification filtrés pour calculer une fréquence de chacun des nucléotides à chaque position des décamères dans les sites de ramification filtrés ;
la normalisation de la fréquence calculée en utilisant une fréquence de fond pour chacun des nucléotides à chaque position des décamères ; et
la construction d'une matrice (m*n) en utilisant la fréquence normalisée pour obtenir la PWM, et dans lequel la construction de la matrice (m*n) comprend la conversion de chacune des fréquences normalisées en valeurs de log impair et la construction de la matrice (m*n) dans l'évaluateur PWM en utilisant les valeurs de log impair ; et
dans lequel l'étape d'évaluation de l'effet de l'au moins un variant candidat sur la région de site accepteur d'épissage pour un nouveau site accepteur d'épissage en cours de création comprend :
la détermination de la présence ou de l'absence d'un point de ramification naturel dans une plage de séquence de 15 nucléotides à 50 nucléotides de la nouvelle région de site accepteur d'épissage étant active pendant l'épissage de pré-ARNm, et
sur la base de la présence ou de l'absence déterminée du point de ramification naturel,
l'évaluation d'une force du point de ramification naturel en utilisant l'évaluateur PWM et l'identification de l'au moins un variant candidat comme causant une maladie sur la base de la force évaluée du point de ramification naturel ; ou
le criblage d'un point de ramification alternatif en utilisant l'évaluateur PWM et la prédiction de l'au moins un candidat comme causant une maladie sur la base de la force évaluée du point de ramification alternatif,
dans lequel le procédé comprend en outre :
pendant l'absence du point de ramification alternatif,
la détermination d'un statut de la région de site accepteur d'épissage naturel, dans lequel le statut comprend une région de site accepteur d'épissage naturel disruptive ou une région de site accepteur d'épissage naturel non disruptive ; et
la prédiction de l'au moins un variant candidat comme causant une maladie ou non causant une maladie sur la base du statut déterminé ; et
la prédiction de la capacité causant une maladie de l'au moins un variant candidat sur la base de l'effet évalué de l'au moins un variant candidat sur l'épissage de pré-ARNm.

2. Procédé mis en œuvre par processeur selon la revendication 1, dans lequel l'évaluateur PWM généré évalue une force de point de ramification sur la base d'un score de seuil, et dans lequel le score de seuil est déterminé en utilisant une pluralité de scores de site de ramification obtenus pour des sites de ramification avec « A » comme point de ramification.

3. Procédé mis en œuvre par processeur selon la revendication 1, dans lequel l'étape d'évaluation de l'effet de l'au moins un variant candidat sur la région de site accepteur d'épissage pour aucun nouveau site accepteur d'épissage en cours de création comprend :
la détermination d'un effet de l'au moins un variant candidat sur le point de ramification naturel, et l'identification d'un niveau de force de site de ramification naturel en utilisant l'évaluateur PWM sur la base de l'effet déterminé ;
le criblage d'une région de site accepteur d'épissage alternative dans une plage de séquences ayant 50 nucléotides en amont et 50 nucléotides en aval de l'au moins un variant candidat et la réalisation d'une comparaison de force de la région de site accepteur d'épissage alternative et de la région de site accepteur d'épissage naturelle affaiblie ; et
la détermination d'une présence d'un nouveau point de ramification en cours de création et la réalisation d'une comparaison de force du nouveau point de ramification et du point de ramification naturel,
dans lequel le procédé comprend en outre :
sur la base du niveau identifié de la force de site de ramification naturel, l'identification de l'au moins un variant candidat comme non causant une maladie ; ou
l'identification de l'au moins un variant candidat comme causant une maladie ou non causant une maladie sur la base d'un point de ramification alternatif criblé dans la séquence 50 nucléotides à 15 nucléotides en amont de la région de site accepteur d'épissage naturel.
dans lequel le procédé comprend en outre :
sur la base de la comparaison de force de région de site accepteur d'épissage alternative et de site accepteur d'épissage naturel affaibli :
la prédiction de l'au moins un variant candidat comme non causant une maladie ; ou
la détermination d'une présence d'un point de ramification naturel dans une plage de séquences de 15 nucléotides à 50 nucléotides de la région de site accepteur d'épissage étant active pendant l'épissage d'ARNm et la comparaison de force du point de ramification naturel avec le seuil prédéfini, dans lequel, sur la base de la présence déterminée et de la comparaison,
la prédiction de l'au moins un variant candidat comme causant une maladie ou non causant une maladie sur la base d'un point de ramification alternatif criblé dans la plage de séquences de 50 nucléotides à 15 nucléotides en amont du site accepteur d'épissage alternatif.

4. Procédé mis en œuvre par processeur selon la revendication 3, comprenant en outre, sur la base de la comparaison de force du nouveau point de ramification et du point de ramification naturel,
la prédiction de l'au moins un variant candidat comme non causant une maladie ; ou
la détermination d'une présence d'un point de ramification naturel dans la plage de 15 nucléotides à 50 nucléotides en amont de la région de site accepteur d'épissage étant active pendant l'épissage de pré-ARNm et la comparaison de force du point de ramification naturel avec le seuil prédéfini.

5. Procédé mis en œuvre par processeur selon la revendication 3, sur la base de la présence déterminée du point de ramification naturel et de la comparaison de force du point de ramification naturel avec le seuil prédéfini, la prédiction de l'au moins un variant candidat comme causant une maladie ou non causant une maladie.

6. Procédé mis en œuvre par processeur selon la revendication 1, dans lequel l'étape d'évaluation de l'effet de l'au moins un variant candidat sur le site de ramification pour le nouveau site accepteur d'épissage en cours de création comprend :
la détermination de la présence d'un point de ramification alternatif dans une plage de séquences ayant 50 nucléotides à 15 nucléotides en amont du nouveau site accepteur d'épissage ; et
la prédiction de l'au moins un variant comme causant une maladie ou non causant une maladie sur la base de la présence du point de ramification alternatif.

7. Procédé mis en œuvre par processeur selon la revendication 1, dans lequel l'étape d'évaluation de l'effet de l'au moins un variant candidat sur le site de ramification pour aucun nouveau site accepteur d'épissage en cours de création comprend :
le criblage d'un point de ramification naturel dans une plage de séquences ayant 50 nucléotides à 15 nucléotides en amont du site accepteur d'épissage naturel ; et
la détermination d'un niveau de force du site de ramification en utilisant l'évaluateur PWM, dans lequel la détermination du niveau de force est due à l'au moins un variant candidat affectant le point de ramification naturel criblé,
dans lequel le procédé comprend en outre :
sur la base du niveau déterminé de force du site de ramification,
la prédiction de l'au moins un variant candidat comme causant une maladie ; ou
la prédiction de l'au moins un variant candidat comme causant une maladie ou non causant une maladie sur la base d'un point de ramification alternatif criblé dans une plage de séquences de 50 nucléotides à 15 nucléotides en amont de la région de site accepteur d'épissage naturel.

8. Système (102) comprenant :
une mémoire stockant des instructions ;
un ou plusieurs processeurs matériels couplés à la mémoire, dans lequel les un ou plusieurs processeurs matériels sont configurés par les instructions pour :
recevoir des informations de position génomique d'au moins un variant candidat de transcrits de gènes et des informations de coordonnées des transcrits de gènes, dans lequel chaque transcrit est représenté comme un ensemble d'un ou plusieurs intervalles non chevauchants, où chaque intervalle non chevauchant est représenté par quatre caractéristiques qui incluent un chromosome sur lequel le transcrit est présent, une coordonnée génomique de départ de l'intervalle non chevauchant, une coordonnée génomique de fin de l'intervalle non chevauchant, et un brin sur lequel le transcrit est présent, **caractérisé en ce que** :
classifier l'au moins un variant candidat dans l'une d'une région de site accepteur d'épissage et d'une région de site de ramification sur la base des informations de coordonnées des transcrits de gènes et des informations de position génomique d'au moins un variant candidat, dans lequel l'au moins un variant candidat est classé
comme se produisant dans la région de site accepteur d'épissage ayant une coordonnée génomique entre 15 nucléotides en amont et 3 nucléotides en aval d'une jonction accepteur d'épissage intron-exon naturel des transcrits de gènes, et
comme se produisant dans la région de site de ramification ayant une coordonnée génomique entre 50 nucléotides et 15 nucléotides en amont d'une jonction accepteur d'épissage naturel des transcrits de gènes ;
évaluer l'effet de l'au moins un variant candidat sur l'épissage de pré-ARNm, sur la base d'une région classée à partir de la classification de l'au moins un variant candidat, dans lequel l'évaluation de l'effet de l'au moins un variant candidat sur l'épissage de pré-ARNm comprend :
l'identification d'un affaiblissement d'un site accepteur d'épissage naturel dans la région classée, dû à l'au moins un variant candidat, en utilisant un score MaxEnt, dans lequel le score MaxEnt est une technique de détermination de force de site d'épissage pour calculer une force ou un affaiblissement du site accepteur d'épissage, et dans lequel le score MaxEnt est attribué sur la base de l'effet de l'au moins un variant candidat sur la région de site accepteur d'épissage naturel affectée ;
la détermination qu'une nouvelle région de site accepteur d'épissage est en cours de création en raison du site accepteur d'épissage naturel affaibli ; et
l'évaluation d'une force d'un point de ramification naturel identifié dans la région classée en utilisant un évaluateur PWM en réponse à la détermination que la nouvelle région de site accepteur d'épissage est en cours de création, dans lequel l'évaluateur PWM est généré en utilisant des sites de ramification humains déterminés expérimentalement, dans lequel la génération de l'évaluateur PWM comprend :
le filtrage des sites de ramification humains déterminés pour décamères ayant « A » en tant que point de ramification ;
l'alignement des sites de ramification filtrés pour calculer une fréquence de chacun des nucléotides à chaque position des décamères dans les sites de ramification filtrés ;
la normalisation de la fréquence calculée en utilisant une fréquence de fond pour chacun des nucléotides à chaque position des décamères ; et
la construction d'une matrice (m*n) en utilisant la fréquence normalisée pour obtenir la PWM, et dans lequel la construction de la matrice (m*n) comprend la conversion de chacune des fréquences normalisées en valeurs de log impair et la construction de la matrice (m*n) dans l'évaluateur PWM en utilisant les valeurs de log impair ; et
dans lequel l'étape d'évaluation de l'effet de l'au moins un variant candidat sur la région de site accepteur d'épissage pour un nouveau site accepteur d'épissage en cours de création comprend :
la détermination de la présence ou de l'absence d'un point de ramification naturel dans une plage de séquences de 15 nucléotides à 50 nucléotides de la nouvelle région de site accepteur d'épissage étant active pendant l'épissage de pré-ARNm, et
sur la base de la présence ou de l'absence déterminée du point de ramification naturel,
l'évaluation d'une force du point de ramification naturel en utilisant l'évaluateur PWM et l'identification de l'au moins un variant candidat comme causant une maladie sur la base de la force évaluée du point de ramification naturel ; ou
le criblage d'un point de ramification alternatif en utilisant l'évaluateur PWM et la prédiction de l'au moins un candidat comme causant une maladie sur la base de la force évaluée du point de ramification alternatif,
dans lequel le procédé comprend en outre :
pendant l'absence du point de ramification alternatif,
la détermination d'un statut de la région de site accepteur d'épissage naturel, dans lequel le statut comprend une région de site accepteur d'épissage naturel disruptive ou une région de site accepteur d'épissage naturel non disruptive ; et
la prédiction de l'au moins un variant candidat comme causant une maladie ou non causant une maladie sur la base du statut déterminé ; et ;
la prédiction de la capacité causant une maladie de l'au moins un variant candidat sur la base de l'effet évalué de l'au moins un variant candidat sur l'épissage de pré-ARNm.

9. Système selon la revendication 8, dans lequel l'évaluation de l'effet de l'au moins un variant candidat sur la région de site accepteur d'épissage pour aucun nouveau site accepteur d'épissage en cours de création comprend :
la détermination d'un effet de l'au moins un variant candidat sur le point de ramification naturel, et l'identification d'un niveau de force de site de ramification naturel en utilisant l'évaluateur PWM sur la base de l'effet déterminé ;
le criblage d'une région de site accepteur d'épissage alternative dans une plage de séquences ayant 50 nucléotides en amont et 50 nucléotides en aval de l'au moins un variant candidat et la réalisation d'une comparaison de force de région de site accepteur d'épissage alternative et de région de site accepteur d'épissage naturel affaibli ; et
la détermination d'une présence d'un nouveau point de ramification en cours de création et la réalisation d'une comparaison de force du nouveau point de ramification et du point de ramification naturel.
dans lequel les un ou plusieurs processeurs matériels sont en outre configurés par les instructions sur la base du niveau identifié de la force de site de ramification naturel pour :
identifier l'au moins un variant candidat comme non causant une maladie ; ou
identifier l'au moins un variant candidat comme causant une maladie ou non causant une maladie sur la base d'un point de ramification alternatif criblé dans la séquence 50 nucléotides à 15 nucléotides en amont de la région de site accepteur d'épissage naturel.
dans lequel les un ou plusieurs processeurs matériels sont en outre configurés par les instructions sur la base de la comparaison de force de région de site accepteur d'épissage alternative et de site accepteur d'épissage naturel affaibli pour :
prédire l'au moins un variant candidat comme non causant une maladie ; ou
déterminer la présence d'un point de ramification naturel dans une plage de séquences de 15 nucléotides à 50 nucléotides de la région de site accepteur d'épissage étant active pendant l'épissage d'ARNm et la comparaison de force du point de ramification naturel avec le seuil prédéfini.
dans lequel les un ou plusieurs processeurs matériels sont en outre configurés par les instructions sur la base de la comparaison de force du nouveau point de ramification et du point de ramification naturel pour :
prédire l'au moins un variant candidat comme non causant une maladie ; ou
déterminer la présence d'un point de ramification naturel dans la plage de 15 nucléotides à 50 nucléotides en amont de la région de site accepteur d'épissage étant active pendant l'épissage d'ARNm et la comparaison de force du point de ramification naturel avec le seuil prédéfini,
comprenant en outre, sur la base de la présence déterminée et de la comparaison, la prédiction de l'au moins un variant candidat comme causant une maladie ou non causant une maladie sur la base d'un point de ramification alternatif criblé dans la plage de séquences de 50 nucléotides à 15 nucléotides en amont du site accepteur d'épissage alternatif,
dans lequel les un ou plusieurs processeurs matériels sont en outre configurés sur la base de la présence déterminée du point de ramification naturel et de la comparaison de force du point de ramification naturel avec le seuil prédéfini pour :
prédire l'au moins un variant candidat comme causant une maladie ou non causant une maladie.

10. Système selon la revendication 8, dans lequel l'évaluation de l'effet de l'au moins un variant candidat sur le site de ramification pour le nouveau site accepteur d'épissage en cours de création comprend :
la détermination de la présence d'un point de ramification alternatif dans une plage de séquences ayant 50 nucléotides à 15 nucléotides en amont du nouveau site accepteur d'épissage ; et
la prédiction de l'au moins un variant comme causant une maladie ou non causant une maladie sur la base de la présence du point de ramification alternatif.
dans lequel les un ou plusieurs processeurs matériels sont en outre configurés par les instructions pour :
évaluer l'effet de l'au moins un variant candidat sur le site de ramification pour aucun nouveau site accepteur d'épissage en cours de création comprend :
le criblage d'un point de ramification naturel dans une plage de séquences ayant 50 nucléotides à 15 nucléotides en amont du site accepteur d'épissage naturel ; et
la détermination d'un niveau de force du site de ramification en utilisant l'évaluateur PWM, dans lequel la détermination du niveau de force est due à l'au moins un variant candidat affectant le point de ramification naturel criblé,
dans lequel les un ou plusieurs processeurs matériels sont en outre configurés sur la base du niveau déterminé de force du site de ramification pour :
prédire l'au moins un variant candidat comme causant une maladie ; ou
prédire l'au moins un variant candidat comme causant une maladie ou non causant une maladie sur la base d'un point de ramification alternatif criblé dans une plage de séquences de 50 nucléotides à 15 nucléotides en amont de la région de site accepteur d'épissage naturel.
